# EUROPÄISCHE PATENTANMELDUNG

(11) **EP 0 400 403 A2**
(43) Veröffentlichungstag der Anmeldung: **05.12.1990**
(21) Anmeldenummer: 90109300.5
(22) Anmeldetag: 17.05.1990
(51) Int. Cl.: C07D 405/04, C07D 413/04, C07D 471/04, C07D 487/04, A01N 43/38, A01N 43/58

(54) **N-Aryl-Stickstoffheterocyclen**

(30) Priorität: 30.05.1989 DE 3917515
(71) Anmelder: BAYER AG, 51368 Leverkusen (DE)
(72) Erfinder: Kunisch, Franz, Dr., D-5068 Odenthal (DE); Arlt, Dieter, Prof., Dr., D-5000 Koeln 80 (DE); Santel, Hans-Joachim, Dr., D-5090 Leverkusen (DE); Lürssen, Klaus, Dr., D-5060 Bergisch-Gladbach 2 (DE); Schmidt, Robert R., Dr., D-5060 Bergisch-Gladbach 2 (DE)

(57) **Zusammenfassung**

Beschrieben werden neue N-Aryl-Stickstoffheterocyclen der Formel (I) 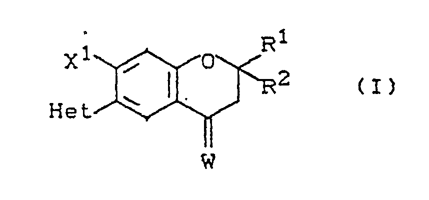 in denen
X¹, R¹, R², Het und W die in der Beschreibung angegebene Bedeutung haben sowie deren Verwendung als Herbizide.

Die N-Aryl-Stickstoffheterocyclen können z.B. erhalten werden, wenn man geeignete Anhydride mit geeigneten Aminen gegebenenfalls in Gegenwart geeigneter Verdünnungs- und/oder Reaktionshilfsmittel umsetzt.

## Beschreibung

Die Erfindung betrifft neue N-Aryl-Stickstoffheterocyclen, mehrere Verfahren sowie neue Zwischenprodukte zu ihrer Herstellung und ihre Verwendung als Herbizide.

Es ist bereits bekannt, daß bestimmte N-Aryl-Stickstoffheterocyclen, wie beispielsweise das 2-tert.-Butyl-4-(2,4-dichlor-5-isopropyloxyphenyl)-5-oxo-1,3,4-oxadiazolin herbizide Eigenschaften besitzen (vgl. US-3 385 862).

Die herbizide Wirksamkeit dieser vorbekannten Verbindungen gegenüber Problemunkräutern ist jedoch ebenso wie ihre Verträglichkeit gegenüber bestimmten Kulturpflanzen nicht in allen Anwendungsgebieten völlig zufriedenstellend.

Es wurden neue N-Aryl-Stickstoffheterocyclen der allgemeinen Formel (I), 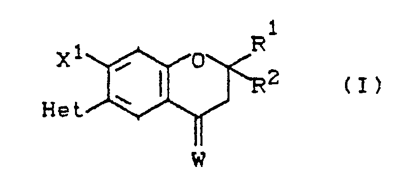 in welcher
X¹ für Wasserstoff oder Halogen steht,
R¹ und R² unabhängig voneinander jeweils für Wasserstoff oder Alkyl stehen und
Het für einen Heterocyclus der Formel 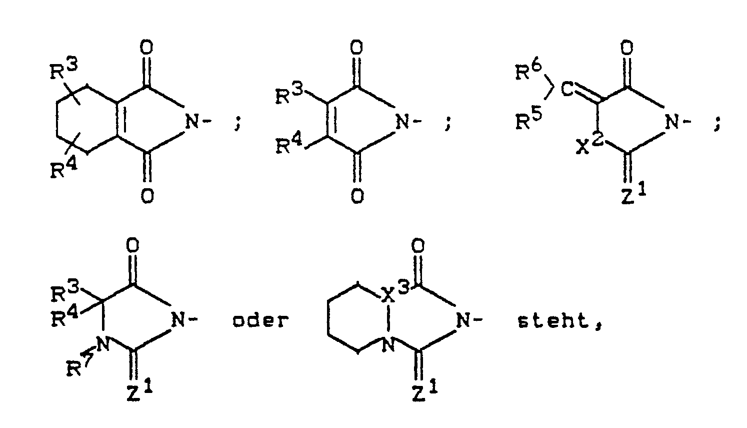 wobei
X² für Sauerstoff, für eine -CH₂-Gruppe oder für eine 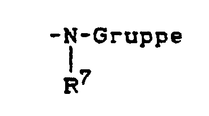 steht,
X³ für Stickstoff oder eine CH-Gruppe steht,
Z¹ für Sauerstoff oder Schwefel steht,
R³ und R⁴ unabhängig voneinander jeweils für Wasserstoff oder Alkyl stehen,
R⁵ und R⁶ entweder unabhängig voneinander jeweils für Wasserstoff oder Alkyl stehen oder gemeinsam für einen zweifach verknüpften Alkandiylrest stehen,
R⁷ für Wasserstoff, Alkyl oder für gegebenenfalls substituiertes Aryl steht und
W für Sauerstoff oder eine NOR⁸-Gruppe steht, wobei
R⁸ für Wasserstoff oder für jeweils gegebenenfalls substituiertes Alkyl, Alkenyl, Alkinyl oder Cycloalkyl steht,
gefunden.

Weiterhin wurde gefunden, daß man die neuen N-Aryl-Stickstoffheterocyclen der allgemeinen Formel (I), 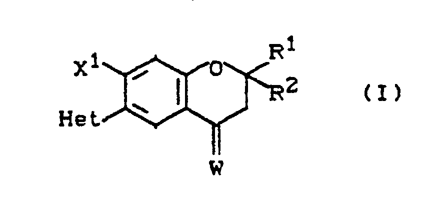 in welcher
X¹ für Wasserstoff oder Halogen steht,
R¹ und R² unabhängig voneinander jeweils für Wasserstoff oder Alkyl stehen und
Het für einen Heterocyclus der Formel 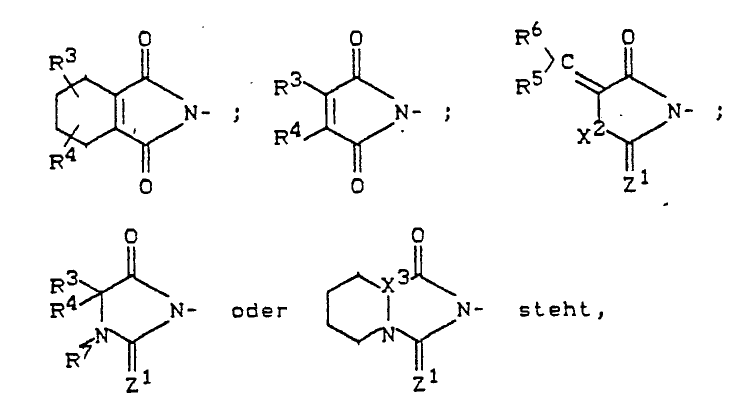 wobei
X² für Sauerstoff, für eine -CH₂-Gruppe oder für eine 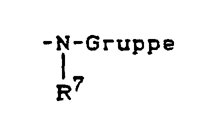 steht,
X³ für Stickstoff oder eine CH-Gruppe steht,
Z¹ für Sauerstoff oder Schwefel steht,
R³ und R⁴ unabhängig voneinander jeweils für Wasserstoff oder Alkyl stehen,
R⁵ und R⁶ entweder unabhängig voneinander jeweils für Wasserstoff oder Alkyl stehen oder gemeinsam für einen zweifach verknüpften Alkandiylrest stehen,
R⁷ für Wasserstoff, Alkyl oder für gegebenenfalls substituiertes Aryl steht und
W für Sauerstoff oder eine NOR⁸-Gruppe steht,
wobei
R⁸ für Wasserstoff oder jeweils gegebenenfalls substituiertes Alkyl, Alkenyl, Alkinyl oder Cycloalkyl steht,
nach einem der im Folgenden beschriebenen Verfahren erhält:
(a) Man erhält N-Aryl-Stickstoffheterocyclen der Formel (Ia), 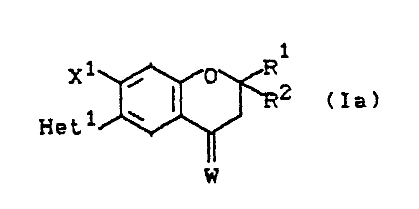 in welcher
   Het¹ für einen Heterocyclus der Formel 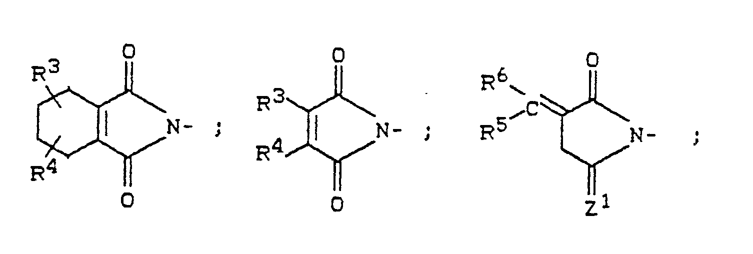 steht, wobei
   Z¹, X¹, W, R¹, R², R³, R⁴, R⁵ und R⁶ die oben angegebene Bedeutung haben,
   wenn man Anhydride der Formel (II), 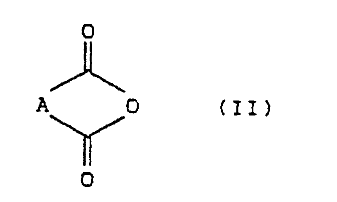 in welcher
   A für einen Rest der Formel 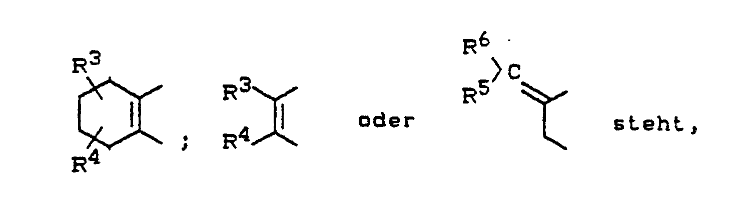 wobei
   R³, R⁴, R⁵ und R⁶ die oben angegebene Bedeutung haben,
   mit Aminen der Formel (III), 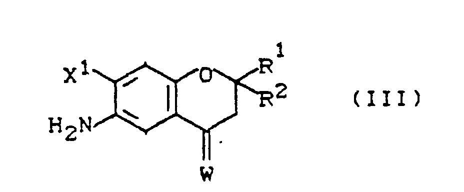 in welcher
   X¹, R¹ und R² und W die oben angegebene Bedeutung haben,
   gegebenenfalls in Gegenwart eines Verdünnungsmittels und gegebenenfalls in Gegenwart eines Reaktionshilfsmittels umsetzt;
(b) man erhält N-Aryl-Stickstoffheterocyclen der Formel (Ib), 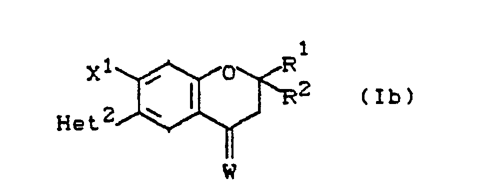 in welcher
   Het² für einen Heterocyclus der Formel 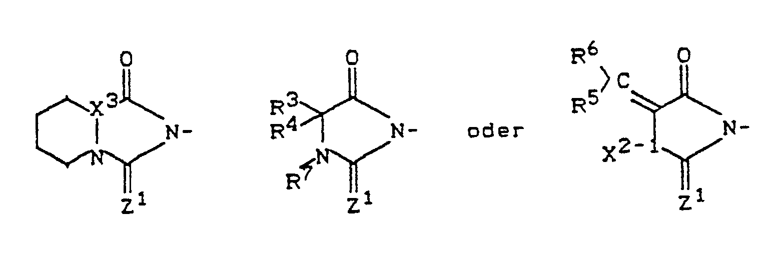 steht, wobei
   X²⁻¹ für Sauerstoff oder eine 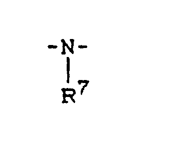 Gruppe steht und
   X¹, W, R¹, R², R³, R⁴, R⁵, R⁶, R⁷, X³ und Z¹ die oben angegebene Bedeutung haben,
   wenn man Carbonsäureester der Formel (IV),
   R⁹-COOR¹⁰ (IV)
   in welcher
   R⁹ für einen Rest der Formel 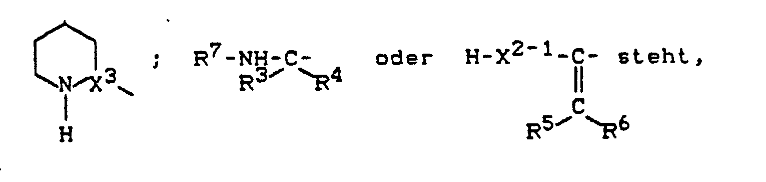 und
   R¹⁰ für Alkyl steht, wobei
   X²⁻¹, X³, R³, R⁴, R⁵, R⁶ und R⁷ die oben angegebene Bedeutung haben,
   oder deren Säureadditionssalze mit Iso(thio)cyanaten der Formel (V), 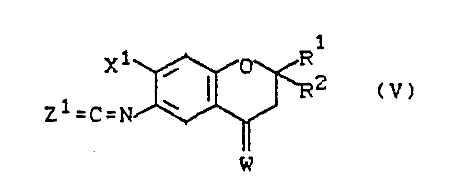 in welcher
   Z¹, X¹, R¹, R² und W die oben angegebene Bedeutung haben,
   gegebenenfalls in Gegenwart eines Verdünnungsmittels und gegebenenfalls in Gegenwart eines Reaktionshilfsmittels umsetzt;
(c) man erhält N-Aryl-Stickstoffheterocyclen der Formel (Ic), 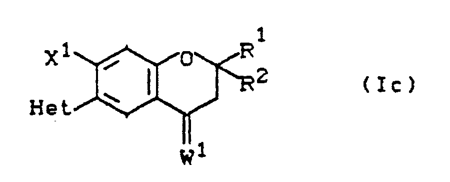 in welcher
   W₁ für eine NOR⁸-Gruppe steht,
   Het für einen Heterocyclus der Formel 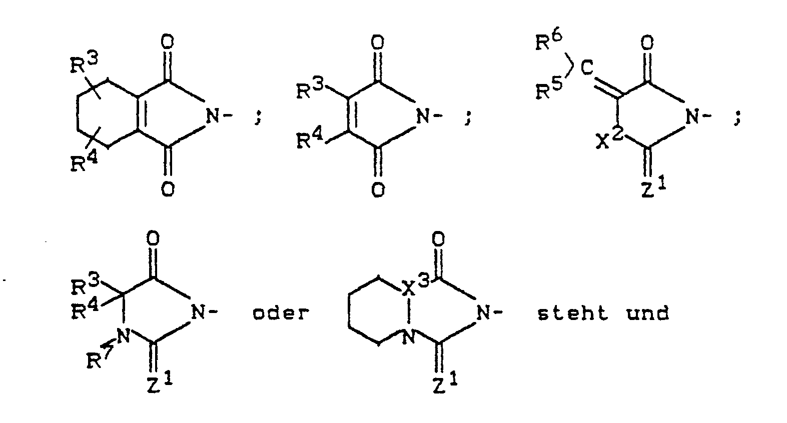 X, X², X³, Z¹, R¹, R², R³, R⁴, R⁵, R⁶, R⁷ und R⁸ die oben angegebene Bedeutung haben,
   wenn man
(c-α) N-Aryl-Stickstoffheterocyclen der Formel (Id) 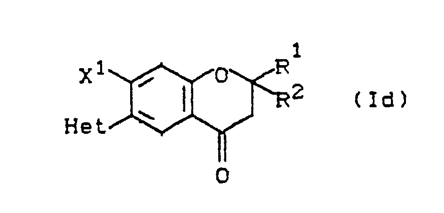 in welcher
   Het, X¹, R¹ und R² die oben angegebene Bedeutung haben
   mit Hydroxylamin-Derivaten der Formel (VI)
   A x H₂N-OR⁸ (VI)
   in welcher
   R⁸ die oben angegebene Bedeutung hat und
   A gegebenenfalls eine anorganische oder organische Säure darstellt, gegebenenfalls in Gegenwart eines Verdünnungsmittels und gegebenfalls in Gegenwart eines Reaktionshilfsmittel umsetzt; oder wenn man
(c-β) N-Aryl-Stickstoffheterocyclen der Formel (Ie) 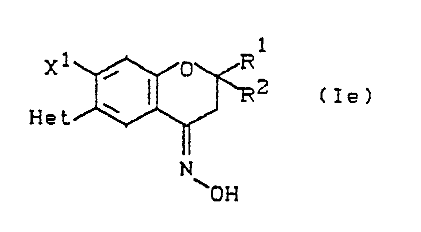 in welcher
   Het, X¹, R¹ und R² die oben angegebene Bedeutung haben
   mit Alkylierungsmitteln der Formel (VII)
   R⁸-E (VII)
   in welcher
   R⁸ die oben angegebene Bedeutung hat und
   E für eine elektronenanziehende Abgangsgruppe steht,
   gegebenenfalls in Gegenwart einer organischen oder anorganischen Base und eines Verdünnungsmittels umsetzt.

Schließlich wurde gefunden, daß die neuen N-Aryl-Stickstoffheterocyclen der allgemeinen Formel (I) ausgezeichnete herbizide Eigenschaften verbunden mit einer sehr guten Nutzpflanzenverträglichkeit besitzen. Die Verbindungen der Formel (I), in denen W für Sauerstoff oder für die NOH-Gruppe steht, sind darüberhinaus auch als wertvolle Zwischenprodukte für erfindungsgemäße N-Aryl-Stickstoffheterocyclen der Formel (I) geeignet.

Überraschenderweise zeigen die erfindungsgemäßen N-Aryl-Stickstoffheterocyclen der Formel (I) eine deutlich bessere herbizide Wirkung und insbesondere im Nachauflauf eine deutlich bessere Nutzzpflanzenverträglichkeit als strukturell ähnliche vrbekannte Verbindungen gleicher Wirkungsrichtung.

Bevorzugt sind Verbindungen der Formel (I), bei welchen
Het für einen Heterocyclus der Formel 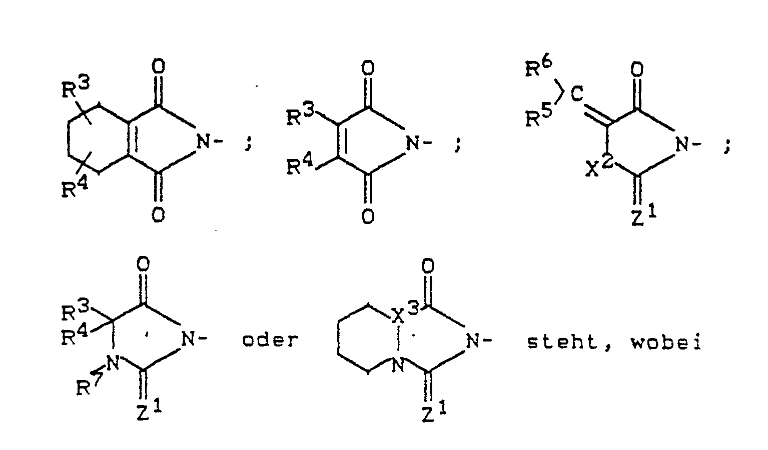 X¹ für Wasserstoff, Fluor, Chlor oder Brom steht und
R¹ und R² unabhänig voneinander jeweils für Wasserstoff oder Alkyl mit 1 bis 4 Kohlenstoffatomen stehen,
X² für Sauerstoff, für eine -CH₂-Gruppe oder für eine 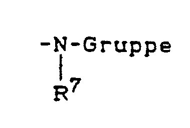 steht,
X³ für Stickstoff oder eine -CH-Gruppe steht,
Z¹ für Sauerstoff oder Schwefel steht,
R³ und R⁴ unabhängig voneinander jeweils für Wasserstoff oder für geradkettiges oder verzweigtes Alkyl mit 1 bis 4 Kohlenstoffatomen stehen,
R⁵ und R⁶ entweder unabhängig voneinander jeweils für Wasserstoff oder für geradkettiges oder verzweigtes Alkyl mit 1 bis 4 Kohlenstoffatomen stehen oder gemeinsam für einen zweifachverknüpften Alkandiylrest mit 2 bis 7 Kohlenstoffatomen stehen,
R⁷ für Wasserstoff, für geradkettiges oder verzweigtes Alkyl mit 1 bis 4 Kohlenstoffatomen oder für gegebenenfalls einfach oder mehrfach, gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen sowie jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen und
W für Sauerstoff oder eine NOR⁸-Gruppe steht,
wobei
R⁸ für Wasserstoff oder für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, Bis-(Alkoxy)alkyl, Bis-(Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen oder für jeweils gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten im Cycloalkylteil jeweils infrage kommen: Halogen sowie jeweils geradkettiges oder verzweigtes Alkyl oder Alkoxy mit jeweils 1 bis 4 Kohlenstoffatomen, R⁸ außerdem für jeweils gegebenenfalls durch Alkyl mit 1 bis 4 Kohlenstoffatomen substituiertes Oxetanylalkyl, Tetrahydrofuranylalkyl oder Tetrahydropyranylalkyl mit jeweils 1 bis 3 Kohlenstoffatomen in den jeweiligen Alkylteilen steht und schließlich für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Arylsubstituenten infrage kommen: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, oder R⁸ für Heteroarylalkyl mit 6 bis 10 Kohlenstoffatomen und einem oder zwei Heteroatomen im Heteroarylteil und 1 bis 4 Kohlenstoffatomen im geradkettigen oder verzweigten Alkylteil, insbesondere Pyridylmethyl, Pyridylethyl, Pyrimidylmethyl oder Pyrimidylethyl, steht.

Besonders bevorzugt ist die Gruppe von Verbindungen der Formel (I), bei welchen
Het für einen Heterocyclus der Formel 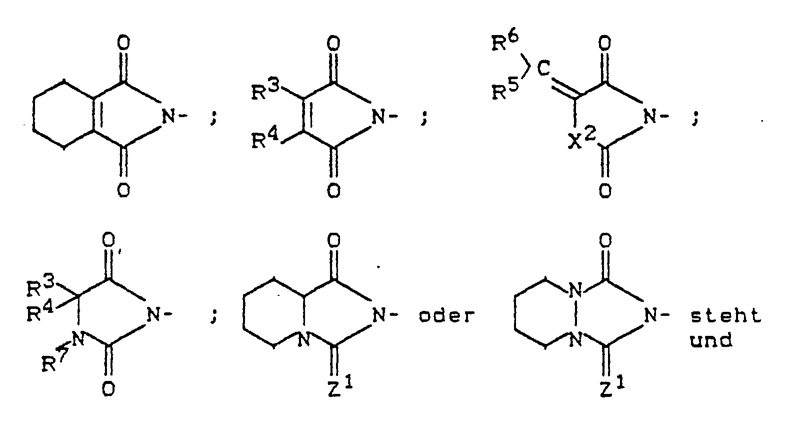 X¹ für Wasserstoff, Fluor oder Chlor steht und
R¹ und R² unabhängig voneinander für Wasserstoff, Methyl, Ethyl, n- oder i-Propyl oder n-, i-, s- oder t-Butyl stehen,
wobei
X² für eine -CH₂-Gruppe oder für eine 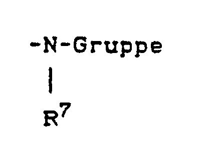 steht,
Z¹ für Sauerstoff oder Schwefel steht,
R³ und R⁴ unabhängig voneinander jeweils für Wasserstoff, Methyl, Ethyl, n- oder i-Propyl stehen,
R⁵ und R⁶ entweder unabhängig voneinander jeweils für Wasserstoff, Methyl, Ethyl, n- oder i-Propyl stehen oder gemeinsam für einen zweifach verknüpften Alkandiylrest mit 2 bis 5 Kohlenstoffatomen stehen,
R⁷ für Wasserstoff, Methyl, Ethyl, n- oder i-Propyl oder für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy oder Trifluormethylthio und
W für Sauerstoff oder für die NOR⁸-Gruppe steht,
wobei
R⁸ für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlen stoffatomen und jeweils 1 bis 8 gleichen oder verschiedenen Halogenatomen, insbesondere Fluor, Chlor oder Brom steht, für jeweils geradkettiges oder verzweigtes Cyanalkyl, Alkoxyalkyl, Alkylthioalkyl, Halogenalkoxyalkyl, Alkoxyalkoxyalkyl, Alkylcarbonylalkyl, Alkoxycarbonylalkyl oder Alkoxyalkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor und Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, für jeweils gegebenenfalls durch Methyl und/oder Ethyl substituiertes Oxetanylmethyl, Oxetanylethyl, Tetrahydrofuranylmethyl, Tetrahydrofuranylethyl, Tetrahydropyranylmethyl oder Tetrahydropyranylethyl steht oder für jeweils gegebenenfalls ein- bis dreifach, gleich oder verschieden substituiertes Benzyl oder Phenylethyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio; oder R⁸ für Pyridylmethyl, Pyridylethyl, Pyrimidylmethyl oder Pyrimidylethyl steht.

Besonders bevorzugt ist auch die Gruppe von Verbindungen der Formel (I), bei welchen W für Sauerstoff oder die NOH-Gruppe steht und die übrigen Reste die oben als besonders bevorzugt angegebene Bedeutung haben.

Ganz besonders bevorzugt ist die Gruppe von Verbindungen der Formel (I), bei welchen
Het für einen Heterocyclus der Formel 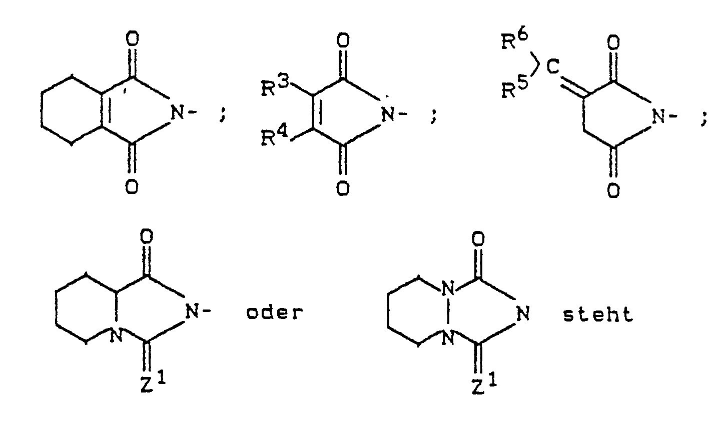 X¹ für Wasserstoff oder Fluor steht und
R¹ und R² unabhängig voneinander für Wasserstoff, Methyl oder Ethyl stehen,
wobei
Z¹ für Sauerstoff oder Schwefel steht,
R³ und R⁴ unabhängig voneinander jeweils für Wasserstoff oder Methyl stehen,
R⁵ und R⁶ entweder unabhängig voneinander jeweils für Wasserstoff oder Methyl oder gemeinsam für einen Ethan-1,2-diylrest, einen Butan-1,4-diylrest oder einen Pentan-1,5-diylrest stehen und
W für Sauerstoff oder eine NOR⁸-Gruppe steht,
wobei
R⁸ für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen, insbesondere Fluor, Chlor oder Brom steht, für jeweils geradkettiges oder verzveigtes Cyanalkyl, Alkoxyalkyl, Alkoxyalkoxyalkyl oder Alkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor und Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, für jeweils gegebenenfalls durch Methyl oder Ethyl substituiertes Oxetanylmethyl, Oxetanylethyl, Tetrahydrofuranylmethyl oder Tetrahydropyranylmethyl steht oder für jeweils gegebenenfalls ein- bis dreifach gleich oder verschieden substituiertes Benzyl oder Phenylethyl steht, wobei als Substituenten jeweils in Frage kommen:
Fluor, Chlor, Brom, Cyano, Nitro, Methyl, Ethyl, n- oder i-propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio.

Ganz besonders bevorzugt ist auch die Gruppe von Verbindungen der Formel (I), in denen W für Sauerstoff oder die -NOH-Gruppe steht und die übrigen Reste die oben als ganz besonders bevorzugt angegebene Bedeutung haben

In Formel (I) ist insbesondere die Bedeutung von 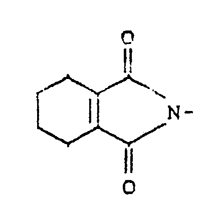 für Het hervorzuheben.

X¹ steht insbesondere für Fluor.

R¹ steht insbesondere für Wasserstoff oder Methyl.

R² steht insbesondere für Wasserstoff.

W steht insbesondere für die Gruppe NOR⁸, wobei R⁸ insbesondere für Methyl, Ethyl, Propargyl, Allyl Methoxycarbonylmethyl oder Ethoxycarbonylmethyl steht.

Verwendet man beispielsweise 3,4,5,6-Tetrahydrophthalsäureanhydrid und 6-Amino-4-methoxyimino-chroman als Ausgangsstoffe, so läßt sich der Reaktionsablauf des erfindungsgemäßen Verfahrens (a) durch das folgende Formelschema darstellen:

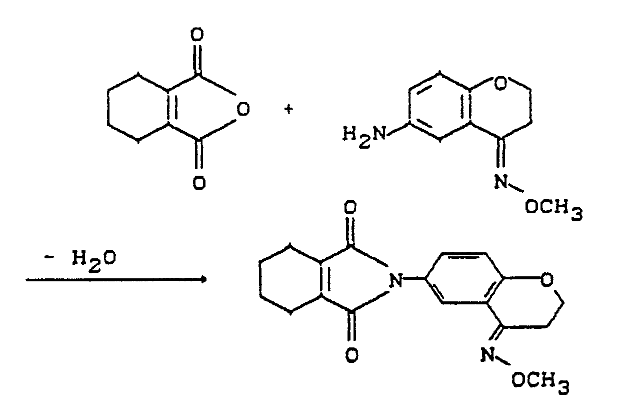

Verwendet man beispielsweise 2-Piperidinylcarbonsäureethylester und 2,3-Dihydro-2-methyl-7-fluor-benzo-4-pyron-6-yl-isocyanat als Ausgangsstoffe, so läßt sich der Reaktionsablauf des erfindungsgemäßen Verfahrens (b) durch das folgende Formelschema darstellen:

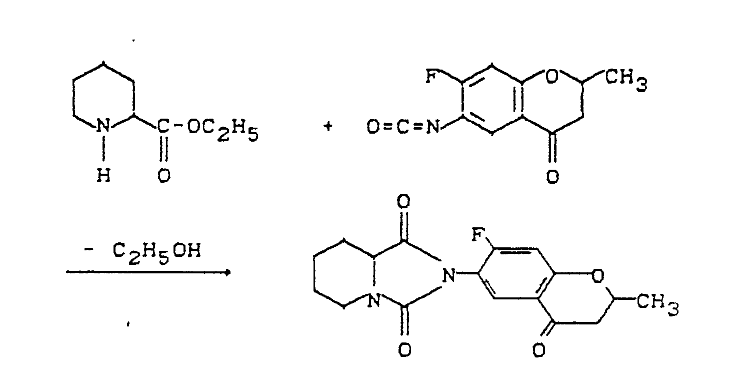

Verwendet man beispielsweise N-(7-Fluor-chroman-4-on-6-yl)-3,4,5,6-tetrahydrophthalimid und O-Methylhydroxylamin-Hydrochlorid als Ausgangsstoffe, so läßt sich der Reaktionsablauf des erfindungsgemäßen Verfahrens (c-α) durch das folgende Formelschema darstellen:

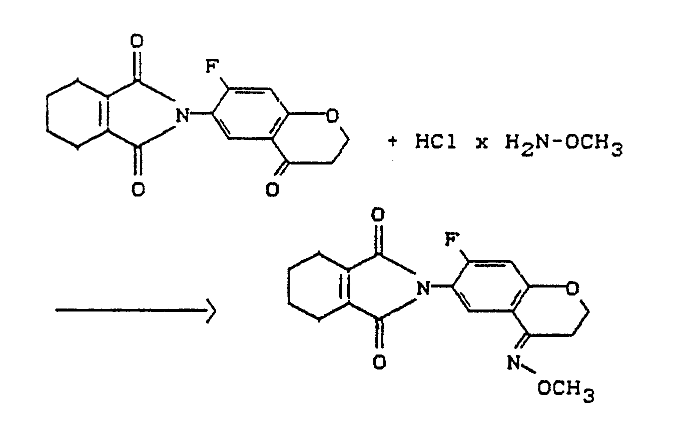

Verwendet man beispielsweise N-(7-Fluor-4-(hydroxyimino)-chroman-6-yl)-3,4,5,6-tetrahydrophthalimid und Bromessigsäureethylester als Ausgangsstoffe, so läßt sich der Reaktionsablauf des erfindungsgemäßen Verfahrens (c-β) durch das folgende Formelschema darstellen:

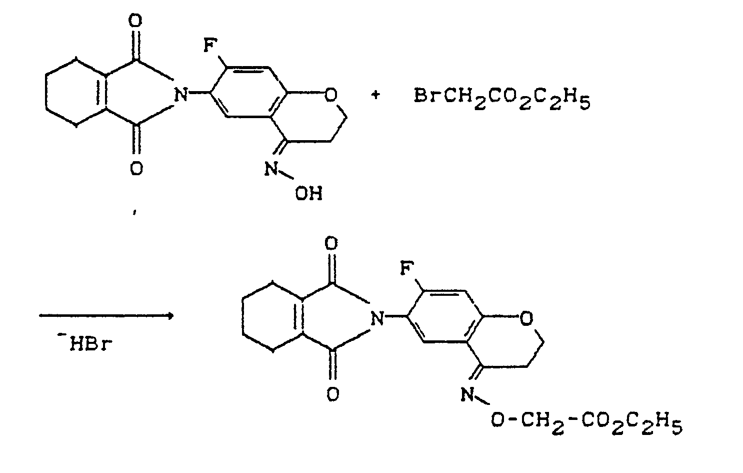

Die zur Durchführung des erfindungsgemäßen Verfahrens (a) als Ausgangsstoffe benötigten Anhydride sind durch die Formel (II) allgemein definiert. In dieser Formel (II) steht A vorzugsweise für einen Rest der Formel 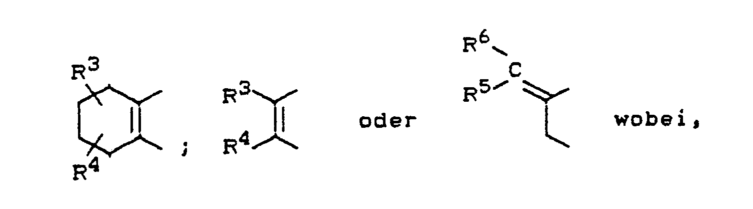 R³, R⁴, R⁵ und R⁶ vorzugsweise für diejenigen Reste stehen, die bereits in Zusammenhang mit der Beschreibung der erfindungsgemäßen Stoffe der Formel (I) als bevorzugt für diese Substituenten genannt wurden.

Die Anhydride der Formel (II) sind bekannt oder erhältlich in Analogie zu bekannten Verfahren (vgl. z.B. Gazz. chim. Ital. 57, 300-311 [1927]; DE-OS 3 644 222; J. org. Chem. 51, 4150-4158 [1986];
Tetrahedron Lett. 25, 6027-6030, [1984]; J. org. Chem. 42, 4162-4164 [1977]; Liebigs Ann. Chem. 1977, 772-790; Tetrahedron 25, 4099-4108 [1969]; JP 43/9046).

Die als Zwischenprodukte benötigten Amine der Formel (III) sind z.T. in der Literatur beschrieben (vgl. z.B. J. Pharm Sci. 72 (1983) II, 1361-1363; US-P 4 115 405 und EP-A228 172; EP-A 230 379; EP-A 225 217; BE 844 943 oder EP-A 1424) oder lassen sich nach bekannten Verfahren herstellen (vgl. z.B. Angew. Chem. 94 (1982), 254-262 sowie Herstellungsbeispiele).

Neu und ebenfalls Gegenstand dieser Anmeldung sind die Amine der allgemeinen Formel (IIIa) 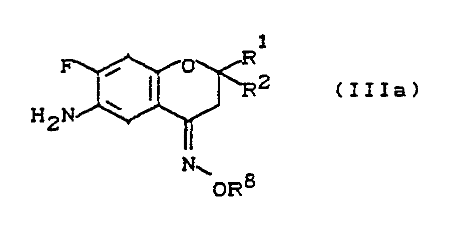 in welcher
R¹ und R² unabhängig voneinander jeweils für Wasserstoff oder Alkyl stehen und
R⁸ für Wasserstoff oder für jeweils gegebenenfalls substituiertes Alkyl, Alkenyl, Alkinyl oder Cycloalkyl steht.

In der Formel (IIIa) stehen die Reste R¹, R² und R⁸ vorzugsweise bzw. besonders bevorzugt für diejenigen Reste, die bereits im Zusammenhang mit der Beschreibung der erfindungsgemäßen Verbindungen der Formel (I) als bevorzugt bzw. besonders bevorzugt für diese Substituenten genannt wurden.

Die neuen Amine der Formel (IIIa) lassen sich beispielsweise analog zu Verfahren (c-α) und (c-β) herstellen.

Die zur Durchführung des erfindungsgemäßen Verfahrens (b) als Ausgangsstoffe benötigten Carbonsäureester sind durch die Formel (IV) allgemein definiert. In dieser Formel (IV) steht R⁹ vorzugsweise für einen Rest der Formel 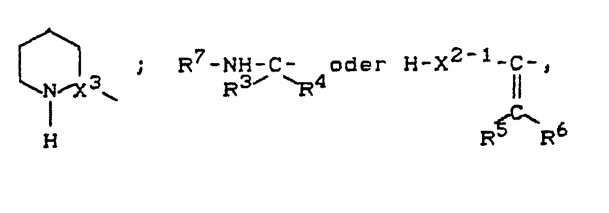 wobei
X²⁻¹ für Sauerstoff oder eine 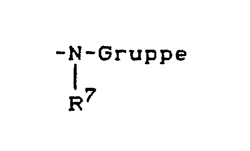 steht und
X³, R³, R⁴, R⁵, R⁶ und R⁷ vorzugsweise für diejenigen Reste stehen, die bereits im Zusammenhang mit der Beschreibung der erfindungsgemäßen Stoffe der Formel (I) als bevorzugt für diese Substituenten genannt wurden.
R¹⁰ steht vorzugsweise für geradkettiges oder verzweigtes Alkyl mit 1 bis 4 Kohlenstoffatomen, insbesondere für Methyl oder Ethyl.

Die Carbonsäureester der Formel (IV) sind allgemein bekannte Verbindungen der organischen Chemie oder erhältlich in Analogie zu bekannten Verfahren (vgl. z.B. US 4 730 006. Pestic. Sci. 16, 277-286 [1985]; Chem. Pharm. Bull. 32, 3934-3944 [1984]; Liebigs Ann. Chem. 1983, 1133-1151; Synthesis 1981, 915-917; Tetrahedron Lett. 22, 2485-2486 [1981]; Chem Ber. 111, 1058-1076 [1978]; Angew. Chem. 89, 344-345 [1977]; Chem. Ber. 110, 942-947 [1977]; Chem. Lett. 1976, 1095-1096; Angew. Chem. 88, 295-296 [1976]; DE 2 058 012; Bull. chem. Soc. Japan 44, 474-477 [1971]; J. chem. Soc. Perkin I, 1987, 877-884; DE 3 702 943; DE 2 331 549; J. Heterocycl. Chem. 6, 181-185, [1969]).

Die zur Durchführung des erfindungsgemäßen Verfahrens (b) weiterhin als Ausgangsstoffe benötigten Iso(thio)cyanate sind durch die Formel (V) allgemein definiert. In dieser Formel (V) stehen R¹, R², X¹, Z¹ und W vorzugsweise für diejenigen Reste, die bereits im Zusammenhang mit der Beschreibung der erfindungsgemäßen Stoffe der Formel (I) als bevorzugt für diese Substituenten genannt wurden.

Die Iso(thio)cyanate der Formel (V) sind erhältlich, in dem man beispielsweise Amine der Formel (III) 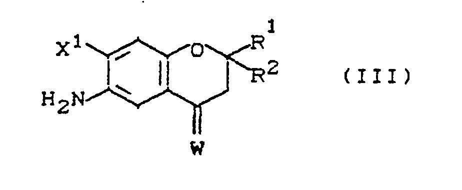 in welcher
X¹, R¹, R² und W die oben angegebene Bedeutung haben,
mit Phosgen, Thiophosgen oder Diphosgen, gegebenenfalls in Gegenwart eines Verdünnungsmittels wie beispielsweise Toluol bei Temperaturen zwischen 20°C und 120°C umsetzt.

Die zur Durchführung des erfindungsgemäßen Verfahrens (c-α) als Ausgangsstoffe benötigten N-Aryl-Stickstoffheterocyclen sind durch die Formel (Id) allgemein definiert.

In dieser Formel (Id) stehen Het, X¹, R¹ und R² vorzugsweise für diejenigen Reste, die bereits im Zusammenhang mit der Beschreibung der erfindungsgemaßen Stoffe der Formel (I) als bevorzugt für diese Substituenten genannt wurden.

Die N-Aryl-Stickstoffheterocyclen der Formel (Id) sind erfindungsgemäße Verbindungen und erhältlich mit Hilfe der erfindungsgemäßen Verfahren (a) oder (b).

Die zur Durchführung des erfindungsgemäßen Verfahrens (c-α) weiterhin als Ausgangsstoffe benötigten O-Alkylhydroxylamin Derivate der Formel (VI) sind allgemein bekannte Verbindungen der organischen Chemie. Die anorganischen oder organischen Säuren werden bei der Beschreibung des Verfahrens (c-α) als Reaktionshilfsmittel näher erläutert.

Die zur Durchführung des erfindungsgemäßen Verfahrens (c-β) als Ausgangsstoffe benötigten N-Aryl-Stickstoffheterocyclen sind durch die Formel (Ie) allgemein definiert. In dieser Formel (Ie) stehen Het, X¹, R¹ und R² vorzugsweise für diejenigen Reste, die bereits im Zusammenhang mit der Beschreibung der erfindungsgemäßen Stoffe der Formel (I) als bevorzugt für diese Substituenten genannt wurden.

Die N-Aryl-Stickstoffheterocyclen der Formel (Ie) sind erfindungsgemäße Verbindungen und erhältlich mit Hilfe der erfindungsgemäßen Verfahren (a), (b) oder (c-α).

Die zur Durchführung des erfindungsgemäßen Verfahrens (c-β) weiterhin als Ausgangsstoffe benötigten Alkylierungsmittel sind durch die Formel (VII) allgemein definiert. In dieser Formel (VII) steht R⁸ vorzugsweise für diejenigen Reste, die bereits im Zusammenhang mit der Beschreibung der erfindungsgemäßen Stoffe der Formel (I) als bevorzugt für diese Substituenten genannt wurden.

E steht für einen bei Alkylierungsmitteln üblichen Abgangsrest, vorzugsweise für Halogen, insbesondere für Chlor, Brom oder Iod, oder für jeweils gegebenenfalls substituiertes Alkylsulfonyloxy mit vorzugsweise 1 bis 4 Kohlenstoffatomen, Alkoxysulfonyloxy mit vorzugsweise 1 bis 4 Kohlenstoffatomen oder Arylsulfonyloxy mit vorzugsweise 6 bis 10 Kohlenstoffatomen, wie insbesondere Methansulfonyloxy, Trifluormethansulfonyloxy, Methoxysulfonyloxy, Ethoxysulfonyloxy oder p-Toluolsulfonyloxy.

Die Alkylierungsmittel der Formel (VII) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Carbonsäuren, wie Essigsäure oder Propionsäure.

Das erfindungsgemäße Verfahren (a) kann gegebenenfalls in Gegenwart eines geeigneten Reaktionshilfsmittels durchgeführt werden. Vorzugsweise verwendet man anorganische oder organische Säuren wie beispielsweise Essigsäure oder p-Toluolsulfonsäure, Anhydride wie beispielsweise Acetanhydrid oder Säurechloride wie Acetylchlorid als Reaktionshilfsmittel. Es ist auch möglich andere übliche wasserabspaltende Mittel wie beispielsweise N,N′-Dicyclohexylcarbodiimid oder übliche Acylierungskatalysatoren, wie beispielsweise 4-(N,N-Dimethylamino)-pyridin als Reaktionshilfsmittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen 20 °C und 180 °C, vorzugsweise bei Temperaturen zwischen 50 °C und 150 °C.

Zur Durchführung des erfindungsgemäßen Verfahrens (a) setzt man pro Mol an Anhydrid der Formel (II) im allgemeinen 1.0 bis 1.5 Mol, vorzugsweise 1.0 bis 1.2 Mol an Amin der Formel (III) und gegebenenfalls 0.01 bis 1.2 Mol, vorzugsweise 0.1 bis 1.0 Mol an Reaktionshilfsmittel ein. Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahren (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Alkohole wie Methanol, Ethanol oder Propanol.

Das erfindungsgemäße Verfahren (b) wird gegebenenfalls in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 150 °C, vorzugsweise bei Temperaturen zwischen 20 °C und 120 °C.

Zur Durchführung des erfindungsgemäßen Verfahrens (b) setzt man pro Mol an Carbonsäureester der Formel (IV) oder eines entsprechenden Säureadditionssalzes im allgemeinen 0.5 bis 5.0 Mol, vorzugsweise 0.8 bis 1.5 Mol an Iso(thio)cyanat der Formel (V) und gegebenenfalls 1.0 bis 10.0 Mol, vorzugsweise 1.0 bis 5.0 Mol an Reaktionshilfsmittel ein. Dabei ist es auch möglich die Iso(thio)cyanate der Formel (V) in einer vorgelagerten Reaktion aus Aminen der Formel (III) und Phosgen, Thiophosgen oder Diphosgen (Cl₃C-O-CO-Cl) direkt im Reaktionsgefäß herstellen und ohne Isolierung im "Eintopfverfahren" mit den Carbonsäureestern der Formel (IV) weiter umzusetzen.

Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (c-α) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -di-ethylether, Ketone, wie Aceton oder Butanon, Nitrile, wie Acetonitril oder Propionitril, Amide, wie Dimethylformamid, Diethylacetamid, N-Methylformanilid, N-Methylpyrrolidon oder Hexamethylphosphorsäuretriamid, Ester, wie Essigsäureethylester oder Sulfoxide, wie Dimethylsulfoxid, Alkohole, wie Methanol, Ethanol oder

Isopropanol, Amine, wie Diethylamin, Diisopropylamin, Diisopropylethylamin und Tri-n-Propylamin oder Pyridin. Es können auch Lösungsmittelgemische eingesetzt werden.

Zur Durchführung des erfindungsgemäßen Verfahrens (c-α) setzt man pro Mol an N-Aryl-Stickstoffheterocyclus der Formel (Id) im allgemeinen 1,0 bis 3,0 Mol, vorzugsweise 1,0 bis 1,5 Mol an Hydroxylamin bzw. O-Alkylhydroxylamin der Formel (VI) ein.

Die Reaktionsdurchführung, Aufarbeitung und Isolierung der Reaktionsprodukte erfolgte nach allgemein üblichen Methoden.

Zur Durchführung des erfindungsgemäßen Verfahrens (c-β) kommen als Verdünnungsmittel inerte organische Lösungsmittel infrage. Vorzugsweise verwendet man aliphatische, alicyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Säuren, wie Essigsäure, Nitrile, wie Acetonitril oder Propionitril, Amide, wie Dimethylformamid, Dimethylacetamid, N-Methylpyrrolidon oder Hexamethylphosphorsäuretriamid. Verwendet man als Reaktionspartner Alkylierungsmittel der Formel (VII) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (c-β) kommen alle üblicherweiseverwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c-β) in einem größeren Bereich variiert werden. Im allgemeinen arbeite man zwischen -20°C und +150°C, vorzugsweise zwischen 0°C und +100°C.

Zur Durchführung des erfindungsgemäßen Verfahrens (c-β) setzt man pro Mol N-Aryl-Stickstoffheterocyclus der Formel (Ie) im allgemeinen jeweils 1,0 bis 20,0 Mol, vorzugsweise jeweils 1,0 bis 15,0 Mol, an Alkylierungsmittel der Formel (VII) und gegebenenfalls 1,0 bis 3,0 Mol, vorzugsweise 1,0 bis 2,0 Mol an Reaktionshilfsmittel, ein. Die Reaktionsdurchführung, Aufarbeitung und Isolierung der Reaktionsprodukte der Formel (Id) erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe der Formel (I) mit besonders gutem Erfolg zur selektiven Bekämpfung von mono- und dikotylen Unkräutern in monokotylen Kulturen, wie beispielsweise Getreide, Mais oder Reis im Vor- und Nachauf laufverfahren einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-EmulsionsKonzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen, in Frage:
z.B Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Anwendung als Herbizide als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Auch Mischungen mit Methyl-5-(2,4-dichlorphenoxy)-2-nitrobenzoat (BIFENOX);
Methyl-2-[4,5-dihydro-4-methyl-4-(1-methylethyl)-5-oxo-1H-imidazol-2-yl]-4(5)-methylbenzoat (IMAZAMETHABENZ);
2-Chlor-N-{[(4-methoxy-6-methyl-1,3,5-triazin-2-yl)-amino]-carbonyl}-benzolsulfonamid (CHLORSULFURON);
2-{[[((4-Methoxy-6-methyl-1,3,5-triazin-2-yl)-amino)-carbonyl]-amino]-sulfonyl}-benzoesäure oder deren Methylester (METSULFURON);
3-[[[[(4-Methoxy-6-methyl-1,3,5-triazin-2-yl)-amino]-carbonyl]-amino]-sulfonyl]-thiophen-2-carbonsäuremethylester (THIAMETHURON);
3-Isopropyl-2,1,3-benzothiadiazin-4-on-2,2-dioxid (BENTAZON);
4-Amino-6-t-butyl-3-ethylthio-1,2,4-triazin-5(4H)-on (ETHIOZIN);
2-[(2-Chlorphenyl)-methyl]-4,4-dimethylisoxazolidin-3-on (DIMETHAZONE);
2,4-Dichlorphenoxyessigsäure (2,4-D);
4-(2,4-Dichlorphenoxy)-buttersäure (2,4-DB);
2-(2,4-Dichlorphenoxy)-propionsäure (2,4-DP);
4-Chlor-2-methylphenoxyessigsäure (MCPA);
2-(2-Methyl-4-chlorphenoxy)-propionsäure (MCPP);
2-[4-(2,4-Dichlorphenoxy)-phenoxy]-propionsäuremethylester (DICLOFOP-METHYL);
2-{4-[-(6-Chlor-2-benzoxazolyl)-oxy]-phenoxy}-propansäure (FENOXAPROP);
N-(1-Ethylpropyl)-3,4-dimethyl-2,6-dinitroanilin (PENDIMETHALIN);
N′-(3-Chlor-4-methylphenyl)-N,N-dimethylharnstoff (CHLORTOLURON);
N′-(4-Isopropylbenzyl)-N,N-dimethylharnstoff (ISOPROTURON);
3,5-Dibrom-4-hydroxybenzonitril (BROMOXYNIL);
3,5-Dijodo-4-hydroxybenzonitril (IOXYNIL);
2,3,3-Trichlorallyldiisopropylthiolcarbamat (TRIALLAT);
2-Ethylamino-4-methylthio-6-t.-butylamino-s-Triazin (TERBUTRYN);
6-Chlor-3-phenylpyridazin-4-yl-S-octyl-thiocarbamat (PYRIDATE);
[(4-Amino-3,5-dichlor-6-fluor-2-pyridinyl)-oxy]-essigsäure (FLUOROXYPYR)
sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogel fraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

### Herstellungsbeispiele

### Beispiel 1

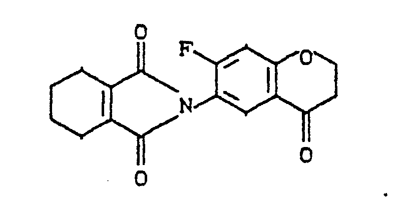

### (Verfahren a)

Eine Mischung aus 2,3 g (15 mMol) 3,4,5,6-Tetrahydrophthalsäureanhydrid und 2,7 g (15 mMol) 6-Amino-7-fluorchroman-4-on wird in 50 ml Eisessig 3 h auf Rückfluß erhitzt. Man gibt auf Eis, saugt den festen Rückstand ab und kristallisiert aus Ethanol um.

Man erhält 2,0 g (42 % d.Theorie) N-(7-Fluor-chroman-4-on-6-yl)-3,4,5,6-tetrahydrophthalimid mit dem Schmelzpunkt 183-189°C.

### Beispiel 2

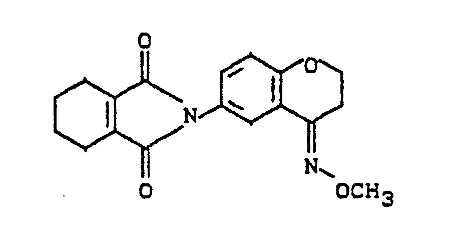

### (Verfahren a)

4,2 g (0,028 Mol) 3,4,5,6-Tetrahydrophthalsäureanhydrid und 5 g (0,028 Mol) 6-Amino-4-methoxyimino-chroman werden mit 0,15 g p-Toluolsulfonsäure in 10 ml Toluol 2,5 h auf Rückfluß erhitzt. Der nach der wässrigen Aufarbeitung erhaltene Rückstand wird chromatographisch an Kieselgel mit dem Laufmittel Petrolether-Essigsäuremethylester = 3:1 gereinigt.

Man erhält 1,7 g (19 % d.Theorie) N-[(4-Methoxyimino)-chroman-6-yl]-3,4,5,6-tetrahydrophthalimid mit dem Schmelzpunkt 141-145°C.

### Beispiel 3

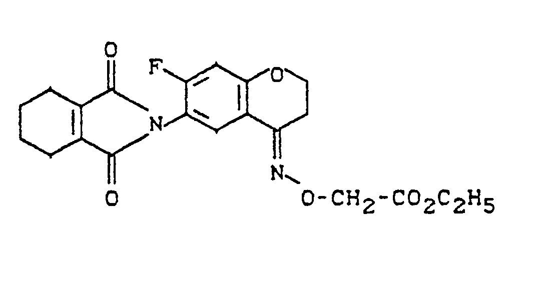

### (Verfahren c-β)

Zu einer Suspension von 300 mg Natriumhydrid (80 % in Öl) in 10 ml Tetrahydrofuran fügt man 2,5 g (7,6 mMol) N-(7-Fluor-4-(hydroxyimino)-chroman-6-yl)-3,4,5,6-tetrahydrophthalimid und 1,2 ml (0,01 Mol) Bromessigsäureethylester und rührt über Nacht bei Rückfluß. Der nach dem wässrigen Aufarbeiten (Extraktion mit Diethylether) erhaltene Rückstand wird an Kieselgel mit dem Laufmittel Petrolether-Essigsäureethylester = 1:1 gereinigt.

Man erhält 1,05 g (33 % d.Theorie) N-[(7-Fluor-4-((ethoxycarbonyl)-methoxy)-imino)-chroman-6-yl]-3,4,5,6-tetrahydrophthalimid mit einem Schmelzpunkt vom 180-182° C.

In entsprechender Weise und gemäß den allgemeinen Angaben zur Herstellung erhält man die folgenden N-Aryl-Stickstoffheterocyclen der allgemeinen Formel (I)

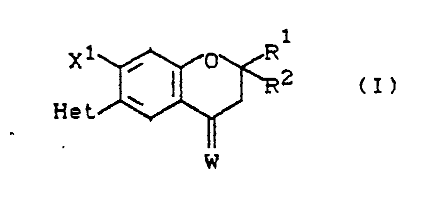

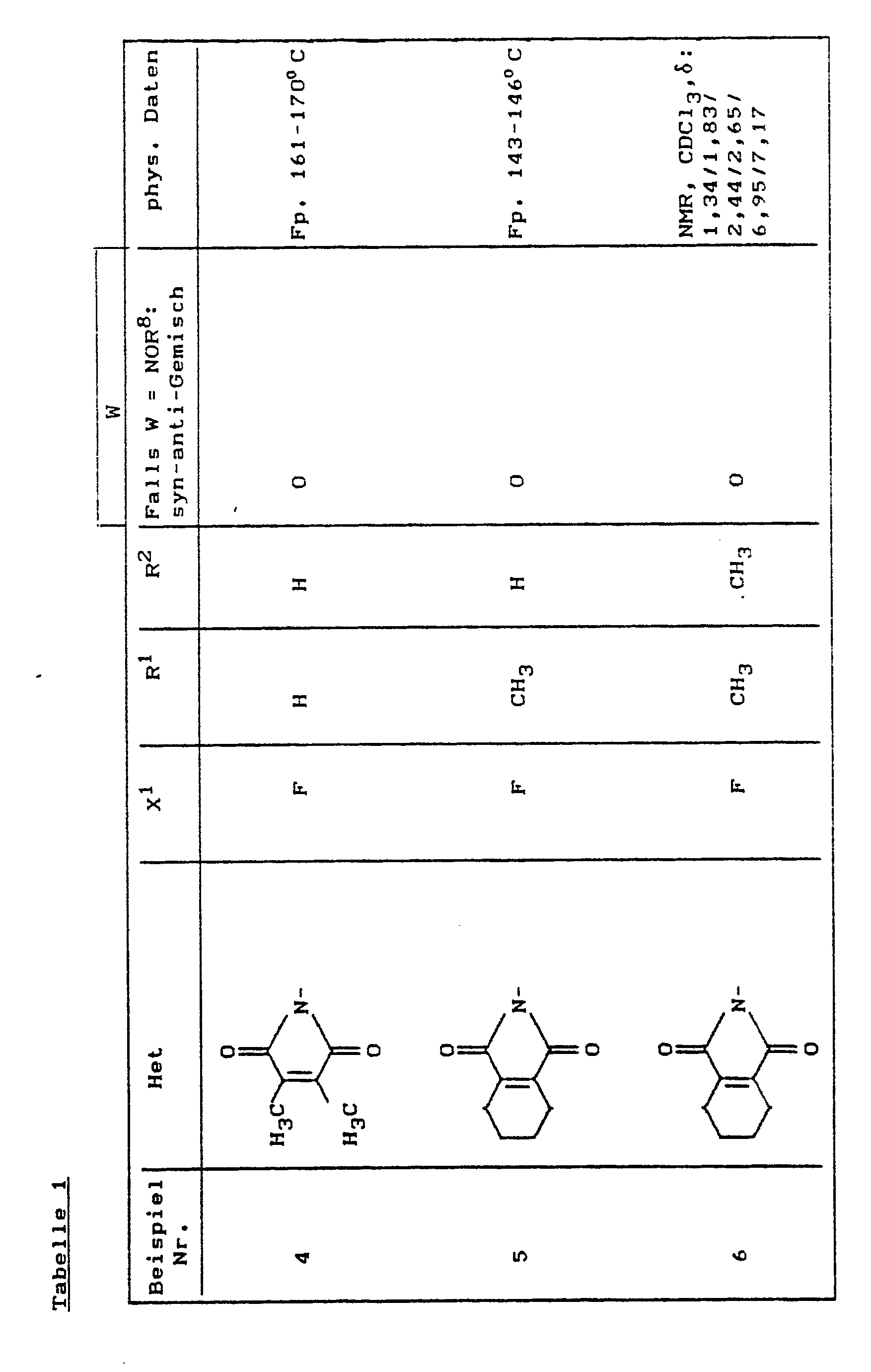

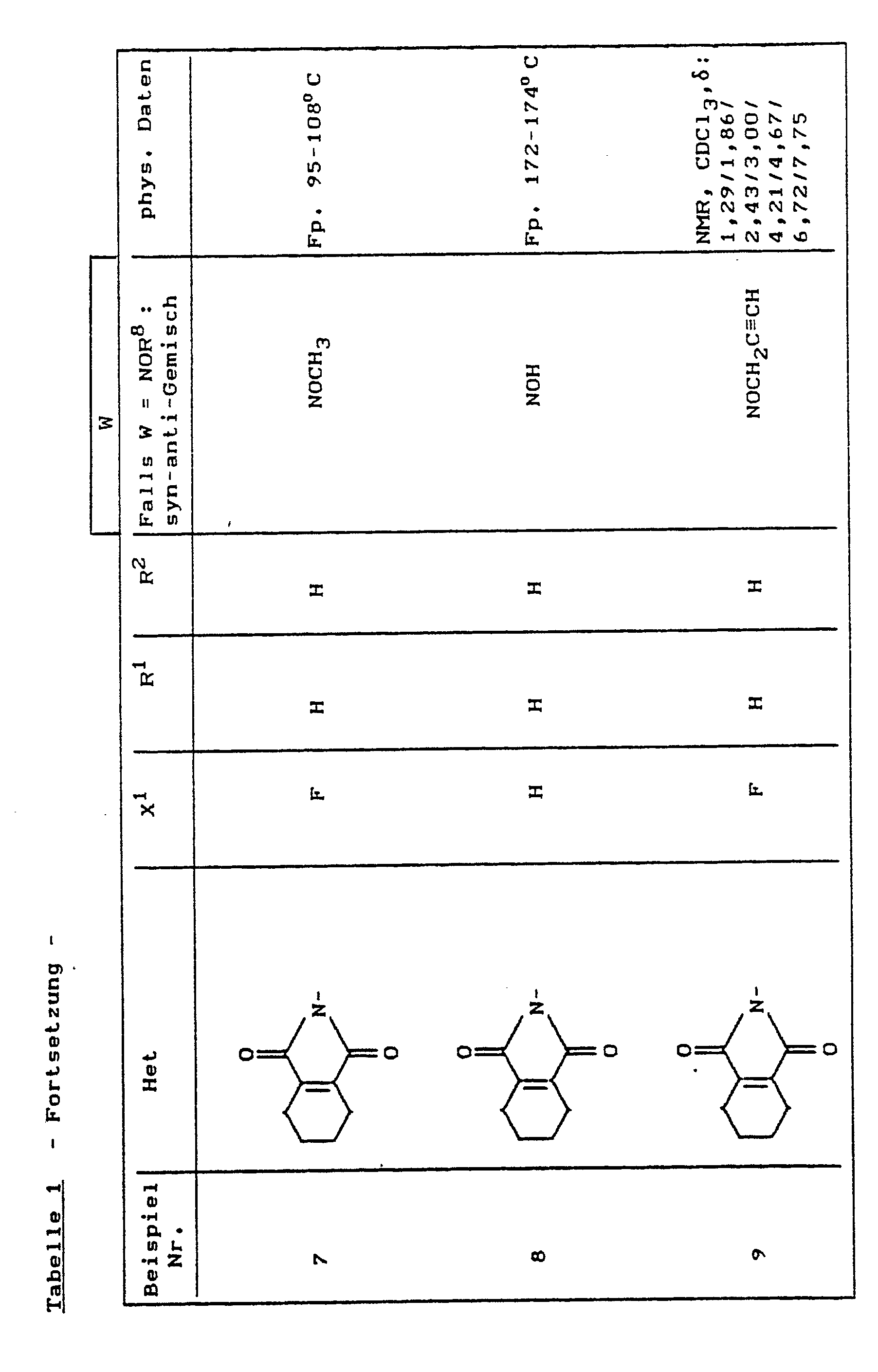

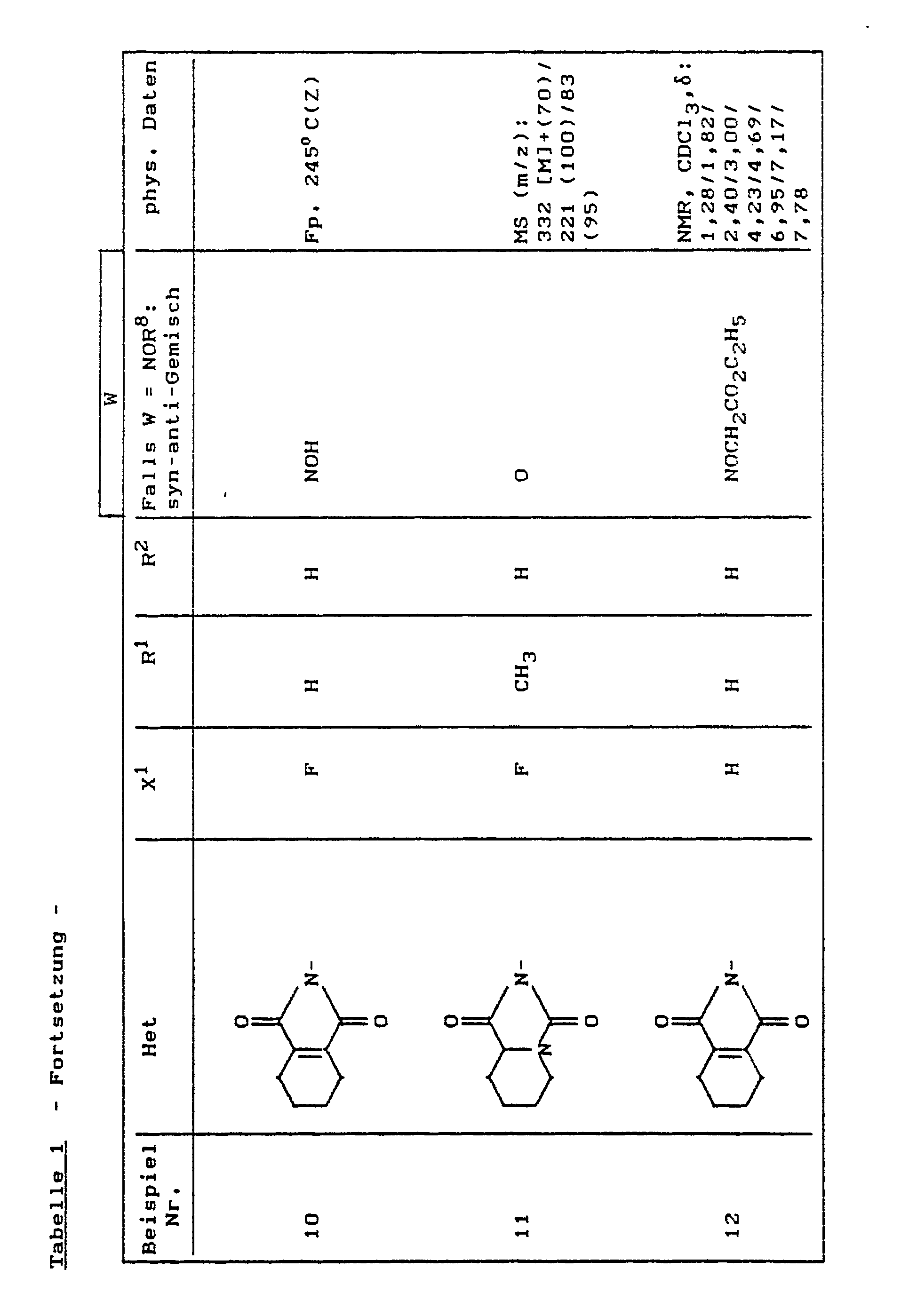

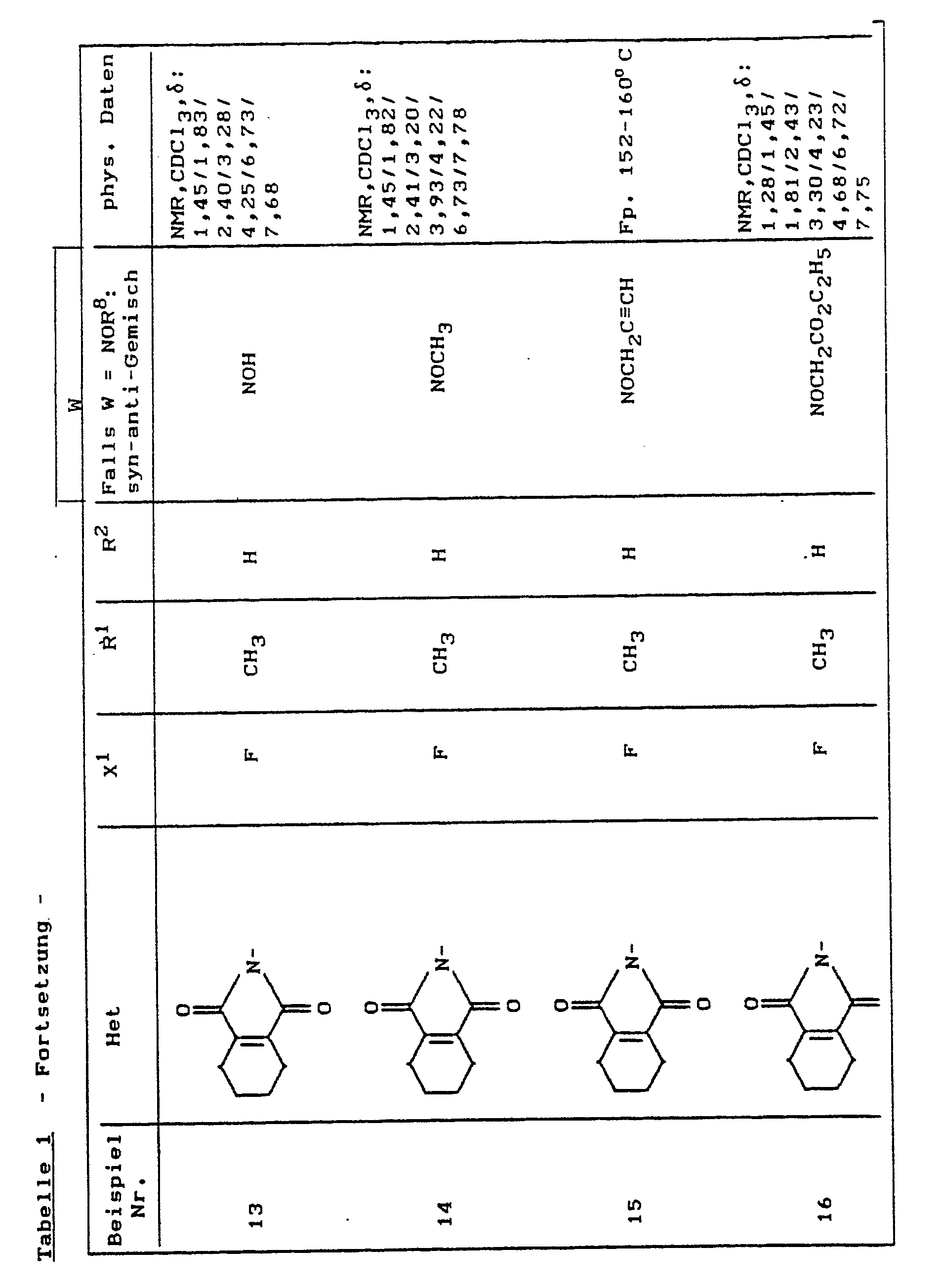

### Herstellung der Ausgangsverbindungen

### Beispiel (III-1)

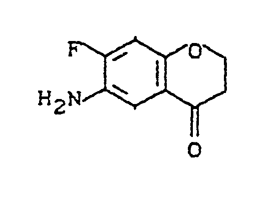

Eine Suspension von 20 g Eisenpulver in 170 ml 5 %iger Essigsäure wird während des Aufheizens auf 90°C portionsweise mit 15 g (0,071 Mol) 6-Nitro-7-fluorchroman-4-on versetzt. Nach 1,5 h Rühren bei 90°C laßt man abkühlen und filtriert vom Rückstand ab. Der Rückstand wird 4 x mit warmen Methanol aufgeschlämmt und die vereinigten Methanol-Filtrate eingeengt. Man erhält 9,7 g (83 % der Theorie) 6-Amino-7-fluor-chroman-4-on mit dem Schmelzpunkt 121-127°C.

### Vorprodukte zur Herstellung vonBeispiel (III-1):

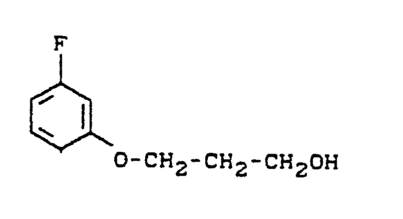

Zu einer Suspension von 13 g (0,43 Mol) Natriumhydrid in 750 ml Dimethylformamid tropft man bei 0°C eine Lösung bestehend aus 50 g (0,446 Mol) 3-Fluorphenol in 100 ml Dimethylformamid. Nach der Zugabe rührt man noch 30 min bei Raumtemperatur nach, kühlt erneut auf 0°C und tropft eine Lösung aus 38 ml (0,45 Mol) 3-Chlorpropanol in 100 ml Dimethylformamid zu. Nach 2,5 h bei 60°C engt man auf ein Volumen von ca. 150 ml ein, verdünnt den Rückstand mit 500 ml Wasser und extrahiert mit Diethylether. Die vereinigten organischen Phasen werden nacheinander mit Wasser, 10 proz. kalter NaOH, Wasser und gesättigter NaCl-Lösung gewaschen und über MgSO₄ getrocknet. Man erhält 72 g (95 % der Theorie) 3-(3-Fluorphenoxy)-propan-1-ol.
IR: 3300-3450 cm⁻¹ 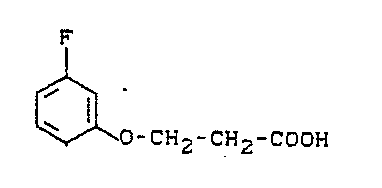 5 g (0,029 Mol) 3-(3-Fluorphenoxy)-propan-1-ol werden in 100 ml Dimethylformamid gelöst. Man fügt 55 g (0,146 Mol) Pyridiniumdichromat (PDC) zu und läßt über Nacht bei Raumtemperatur rühren. Die Reaktionslösung wird zu 300 ml Wasser gegeben und mit Diethylether extrahiert. Die organischen Phasen werden mit Wasser und gesättigter NaCl-Lösung gewaschen, getrocknet und eingeengt. Man erhält 3,9 g (73 % der Theorie) 3-(3-Fluorphenoxy)-propancarbonsäure.
MS: m/z (% rel.Int.): 184 [M⁺] (25), 112 (100)

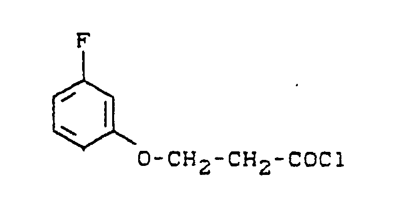

Zu 10 g (0,054 Mol) 3-(3-Fluorphenoxy)-propancarbonsäure in 100 ml Benzol tropft man 11 ml (0,13 Mol) Oxalsäuredichlorid und erhitzt 4 h bei Raumtemperatur. Nach Beendigung der Gasentwicklung wird eingeengt. Man erhält 10,5 g (100 % d.Theorie) 3-(3-Fluorphenoxy)-propancarbonsäurechlorid.
IR: 1795 cm⁻¹

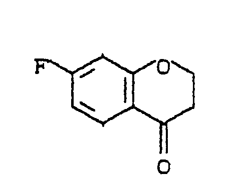

Zu einer Suspension von 4,3 g (0,03 Mol) Aluminiumtrichlorid in 40 ml absolutem 1,2-Dichlorethan tropft man eine Lösung von 5 g (0,025 Mol) 3-(3-Fluorphenoxy)-propancarbonsäurechlorid in 10 ml absolutem 1,2-Dichlorethan. Man rührt 1 h bei Raumtemperatur, gießt dann den Ansatz zu 150 ml Eiswasser und fügt solange konz. HCl zu bis sich der enstandene Niederschlag aufgelöst hat. Die wässrige Phase wird mit CH₂Cl₂ extrahiert und die vereinigten organischen Phasen mit H₂O und gesättigter NaCl-Lösung gewaschen. Nach Trocknen und Einengen wird der Rückstand aus Isopropanol umkristallisiert; es werden 3,5 g (84 % der Theorie) 7-Fluor-chroman-4-on mit einem Schmelzpunkt von 58-60,5°C erhalten.

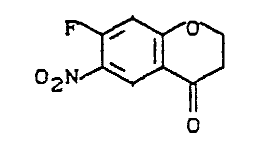

Zu einer Lösung von 25 g (0,15 Mol) 7-Fluor-chroman-4-on in 45 ml konz. Schwefelsäure tropft man bei -10 bis -5°C eine Lösung bestehend aus 6,5 ml konz. Salpetersäure und 13 ml konz. Schwefelsäure zu. Nach beendeter Zugabe läßt man noch 30 min bei 0°C nachrühren und gibt die Reaktionsmischung unter Rühren zu Eiswasser. Der nach dem Absaugen erhaltene Feststoff ergibt nach Umkristallisation aus 300 ml Ethanol 19 g (60 % der Theorie) 6-Nitro-7-fluor-chroman-4-on mit dem Schmelzpunkt 154-155°C.

### Beispiel (III-2)

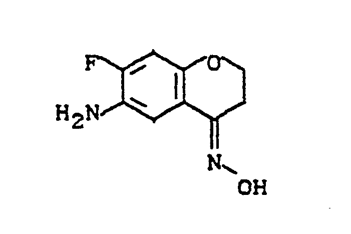

10 g (0,055 Mol) 6-Amino-7-fluor-chroman-4-on und 3,9 g (0,055 Mol) Hydroxylaminhydrochlorid werden in 60 ml Ethanol 1,5 h bei Raumtemperatur umgesetzt. Nach dem Erkalten saugt man den Feststoff ab, wäscht mit Wasser nach und erhält nach Trocknen 9,2 g (87 % der Theorie) 6-Amino-7-fluor-4-hydroxyimino-chroman mit dem Zersetzungspunkt von 234°C.

### Beispiel (III-3)

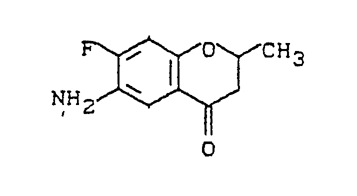

10 g (0,04 Mol) 2-Methyl-6-nitro-7-fluor-chroman-4-on werden in 150 ml Tetrahydrofuran gelöst und nach Zugabevon 5 g Raney-Nickel 2 h bei Raumtemperatur und 40-50 bar Wasserstoff hydriert. Man filtriert vom Katalysator ab, wäscht mit Tetrahydrofuran nach und erhält nach Einengen des Filtrats 7,6 g (89 % der Theorie) 2-Methyl-6-amino-7-fluor-chroman-4-on mit dem Schmelzpunkt 126-135°C.

### Vorprodukte zur Herstellung von Beispiel (III-3):

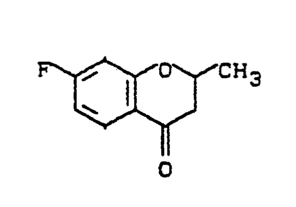

Eine Mischung aus 56 g (0,05 Mol) 3-Fluorphenol und 172 g (2 Mol) Crotonsäure in 1120 ml Methansulfon säure wird 20 h bei 90-95°C umgesetzt. Nach dem Abkühlen gießt man die Reaktionsmischung in 4 l Eiswasser ein, extrahiert 3 x mit je 800 ml Diethylether und wäscht die gesammelten organischen Phasen nacheinander 3 x mit 500 ml Wasser, 4 x mit 500 ml kalter NaOH, 2 x mit 500 ml Wasser und 500 ml mit gesättigter NaCl-Lösung. Nach dem Trocknen über MgSO₄ und Einengen erhält man 43 g (52 % der Theorie) 2-Methyl-7-fluor-chroman-4-on.
IR (CH₂Cl₂): 1695, 1610, 1595 cm⁻¹

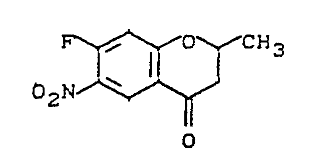

Zu 45 g (0,25 Mol) 2-Methyl-7-fluor-chroman-4-on in 76 ml konz. H₂SO₄ tropft man bei 0°C eine Mischung aus 15,1 ml (0,38 mol) konz. HNO₃ und 32,4 ml konz. H₂SO₄; nach der Zugabe rührt man noch 20 min bei 0°C nach. Die Reaktionsmischung wird in 300 ml Eiswasser eingegossen und die wässrige Phasen 4 x mit Ethylacetat extrahiert. Nach Waschen der organischen Phasen mit Wasser und Trocknen werden 26 g (46 % d.Theorie) 2-Methyl-6-nitro-7-fluor-chroman-4-on erhalten.
¹H-NMR (CDCl₃):
δ = 1,60 (d,3H); 2,80 (2H); 4,78 (m,1H); 6,87 (d,J = 11,5 Hz,1H); 8,68 (d,J = 9 Hz,1H)

### Beispiel (III-4)

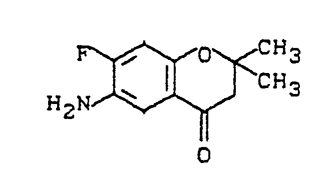

Zu einer Suspension von 15 g (0,27 Mol) Eisenpulver in 150 ml 5 %iger Essigsäure gibt man portionsweise 15 g (0,063 Mol) 2,2-Dimethyl-6-nitro-7-fluor-chroman-4-on und rührt 1 h bei 90°C. Der nach dem Abkühlen und Absaugen erhaltene Rückstand wird 3 x mit Methanol aufschlämmt; die vereinigten Filtrate ergeben nach Einengen 9,6 g (74 % der Theorie) 2,2-Dimethyl-6-amino-7-fluor-chroman-4-on.

### Vorprodukte zur Herstellung von Beispiel (III-4):

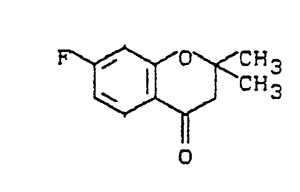

Eine Mischung aus 20,0 g (0,178 Mol) 3-Fluorphenol, 71,0 g (0,71 Mol) 2,2-Dimethylacrylsäure und 400 ml Methansulfonsäure wird 20 h auf 90-95°C erhitzt. Man kühlt die Reaktionsmischung auf 0°C, gießt sie zu 600 g Eis in 600 ml Wasser und extrahiert 3 x mit je 350 ml Diethylether. Die vereinigten organischen Phasen werden nacheinander 3 x mit 150 ml Wasser, 4 x mit 180 ml kalter Natronlauge, 2 x mit 200 ml Wasser und zuletzt mit 250 ml gesättigter NaCl-Lösung gewaschen. Das nach dem Trocknen über MgSO₄ und Einengen erhaltene Produkt wird säulenchromatographisch an Kieselgel, Petrolether-Essigsäure-ethylester (7:1) gereinigt. Man erhält 10 g (29 % der Theorie) 2,2-Dimethyl-7-fluor-chroman-4-on.
MS: m/z (% rel.Int.): 194[M+] (25); 179 (100); 152 (14); 109 (27).

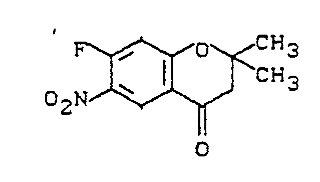

Bei -5 bis 0°C tropft man eine Mischung aus 7,5 ml konz. Salpetersäure und 16 ml konz. Schwefelsäure zu einer Lösung aus 23,5 g (0,121 Mol) 2,2-Dimethyl-7-fluor-chroman-4-on in 36 ml konz. Schwefelsäure. Nach beendeter Zugabe rührt man 1,5 h bei 0°C nach, gibt die Reaktionsmischung auf Eis und saugt den Niederschlag ab. Nach Trocknen werden 27 g (100 % der Theorie) 2,2-Dimethyl-6-nitro-7-fluor-chroman-4-on mit dem Schmelzpunkt 131-144°C erhalten.

### Anwendungsbeispiele

In den folgenden Anwendungsbeispielen wurde die nachstehend aufgeführte Verbindung als Vergleichsubstanz eingesetzt: 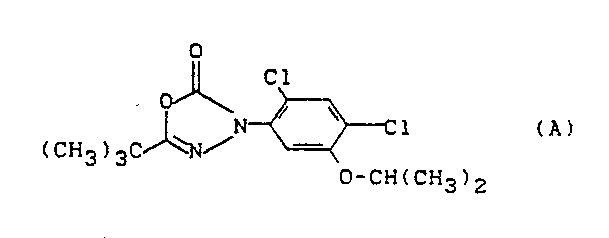 (bekannt aus US-P 3 385 862)

### Beispiel A

| Pre-emergence-Test | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigt z.B. die Verbindung gemäß Herstellungsbeispiel (7) bei vergleichbarer Nutzpflanzenselektivität eine deutlich bessere herbizide Wirkung bei der Bekämpfung von dikotylen Unkräutern als die Vergleichssubstanz (A).

### Beispiel B

| Post-emergence-Test | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigt z.B. die Verbindung gemäß Herstellungsbeisiel (7) eine deutlich bessere Nutzpflanzuenverträglichkeit und eine deutlich bessere herbizide Wirkung als die Vergleichssubstanz (A). Weiterhin zeigen in diesem Test die Verbindungen (9), (12), (14) und (15) eine sehr gute selektive herbizide Wirkung.

## Patentansprüche

1. N-Aryl-Stickstoffheterocyclen der allgemeinen Formel (I), 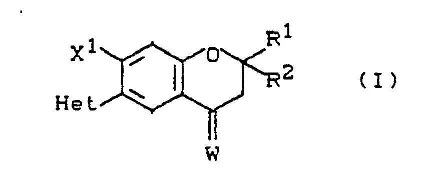 in welcher
X¹ für Wasserstoff oder Halogen steht,
R¹ und R² unabhängig voneinander jeweils für Wasserstoff oder Alkyl stehen und
Het für einen Heterocyclus der Formel 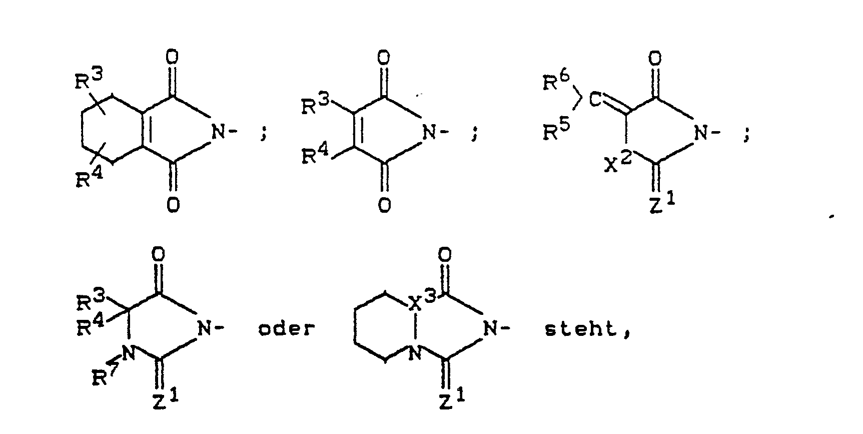 wobei
X² für Sauerstoff, für eine -CH₂-Gruppe oder für eine 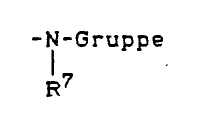 steht,
X³ für Stickstoff oder eine CH-Gruppe steht,
Z¹ für Sauerstoff oder Schwefel steht,
R³ und R⁴ unabhängig voneinander jeweils für Wasserstoff oder Alkyl stehen,
R⁵ und R⁶ entweder unabhängig voneinander jeweils für Wasserstoff oder Alkyl stehen oder gemeinsam für einen zweifach verknüpften Alkandiylrest stehen,
R⁷ für Wasserstoff, Alkyl oder für gegebenenfalls substituiertes Aryl steht und
W für Sauerstoff oder eine -NOR⁸-Gruppe steht,
wobei
R⁸ für Wasserstoff oder für jeweils gegebenenfalls substituiertes Alkyl, Alkenyl, Alkinyl oder Cycloalkyl steht.

2. N-Aryl-Stickstoffheterocyclen der Formel (I) gemäß Anspruch 1, in welcher
Het für einen Heterocyclus der Formel 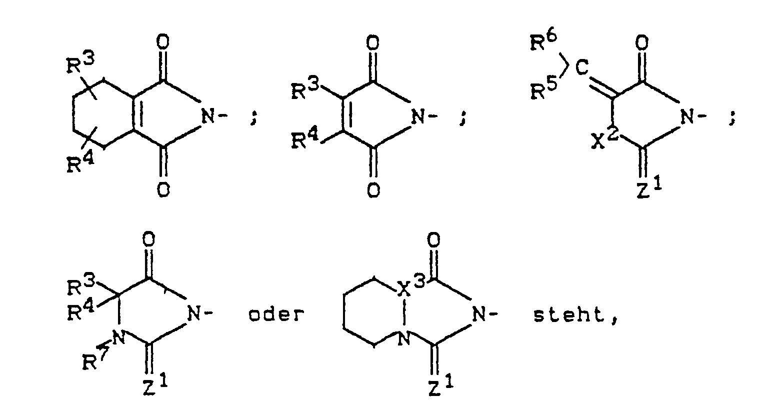 X¹ für Wasserstoff, Fluor, Chlor oder Brom steht und
R¹ und R² unabhänig voneinander jeweils für Wasserstoff oder Alkyl mit 1 bis 4 Kohlenstoffatomen stehen,
wobei
X² für Sauerstoff, für eine -CH₂-Gruppe oder für eine 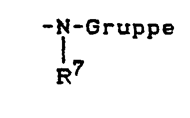 steht,
X³ für Stickstoff oder eine -CH-Gruppe steht,
Z¹ für Sauerstoff oder Schwefel steht,
R³ und R⁴ unabhängig voneinander jeweils für Wasserstoff oder für geradkettiges oder verzweigtes Alkyl mit 1 bis 4 Kohlenstoffatomen stehen,
R⁵ und R⁶ entweder unabhängig voneinander jeweils für Wasserstoff oder für geradkettiges oder verzweigtes Alkyl mit 1 bis 4 Kohlenstoffatomen stehen oder gemeinsam für einen zweifachverknüpften Alkandiylrest mit 2 bis 7 Kohlenstoffatomen stehen,
R⁷ für Wasserstoff, für geradkettiges oder verzweigtes Alkyl mit 1 bis 4 Kohlenstoffatomen oder für gegebenenfalls einfach oder mehrfach, gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen sowie jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen und
W für Sauerstoff oder eine NOR⁸-Gruppe steht,
wobei
R⁸ für Wasserstoff oder für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, Bis-(Alkoxy)alkyl, Bis-(Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen oder für jeweils gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten im Cycloalkylteil jeweils infrage kommen: Halogen sowie jeweils geradkettiges oder verzweigtes Alkyl oder Alkoxy mit jeweils 1 bis 4 Kohlenstoffatomen, R⁸ außerdem für jeweils gegebenenfalls durch Alkyl mit 1 bis 4 Kohlenstoffatomen substituiertes Oxetanylalkyl, Tetrahydrofuranylalkyl oder Tetrahydropyranylalkyl mit jeweils 1 bis 3 Kohlenstoffatomen in den jeweiligen Alkylteilen steht und schließlich für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Arylsubstituenten infrage kommen: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, oder R⁸ weiterhin für Heteroarylalkyl mit 6 bis 10 Kohlenstoffatomen und einem oder zwei Heteroatomen im Heteroarylteil und 1 bis 4 Kohlenstoffatomen im geradkettigen oder verzweigten Alkylteil steht.

3. N-Aryl-Stickstoffheterocyclen der Formel (I) gemäß Anspruch 1,
in welcher
Het für einen Heterocyclus der Formel 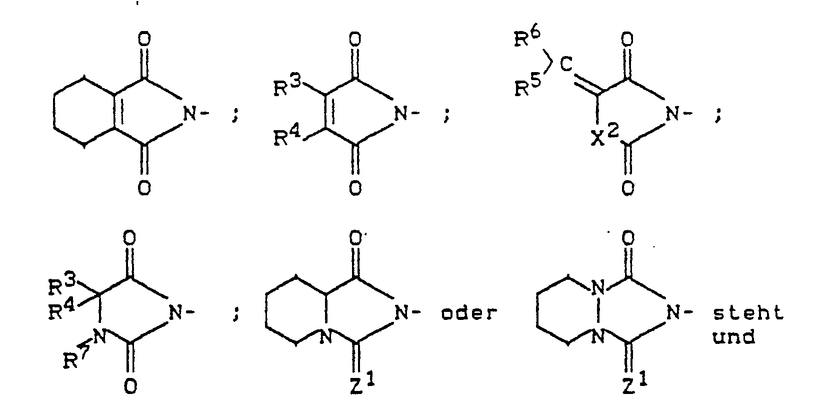 X¹ für Wasserstoff, Fluor oder Chlor steht und
R¹ und R² unabhängig voneinander für Wasserstoff, Methyl, Ethyl, n- oder i-Propyl oder n-, i-, s- oder t-Butyl stehen,
wobei
X² für eine -CH₂-Gruppe oder für eine 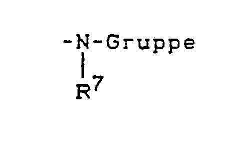 steht,
Z¹ für Sauerstoff oder Schwefel steht,
R³ und R⁴ unabhängig voneinander jeweils für Wasserstoff, Methyl, Ethyl, n- oder i-Propyl stehen,
R⁵ und R⁶ entweder unabhängig voneinander jeweils für Wasserstoff, Methyl, Ethyl, n- oder i-Propyl stehen oder gemeinsam für einen zweifach verknüpften Alkandiylrest mit 2 bis 5 Kohlenstoffatomen stehen,
R⁷ für Wasserstoff, Methyl, Ethyl, n- oder i-Propyl oder für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy oder Trifluormethylthio und
W für Sauerstoff oder für die NOR⁸-Gruppe steht,
wobei
R⁸ für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 8 gleichen oder verschiedenen Halogenatomen, insbesondere Fluor, Chlor oder Brom steht, für jeweils geradkettiges oder verzweigtes Cyanalkyl, Alkoxyalkyl, Alkylthioalkyl, Halogenalkoxyalkyl, Alkoxyalkoxyalkyl, Alkylcarbonylalkyl, Alkoxycarbonylalkyl oder Alkoxyalkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor und Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, für jeweils gegebenenfalls durch Methyl und/oder Ethyl substituiertes Oxetanylmethyl, Oxetanylethyl, Tetrahydrofuranylmethyl, Tetrahydrofuranylethyl, Tetrahydropyranylmethyl oder Tetrahydropyranylethyl steht oder für jeweils gegebenenfalls ein- bis dreifach, gleich oder verschieden substituiertes Benzyl oder Phenylethyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio; oder R⁸ für Pyridylmethyl, Pyridylethyl, Pyrimidylmethyl oder Pyrimidylethyl steht.

4. N-Aryl-Stickstoffheterocyclen der Formel (I) gemäß Anspruch 1, in welcher
Het für einen Heterocyclus der Formel 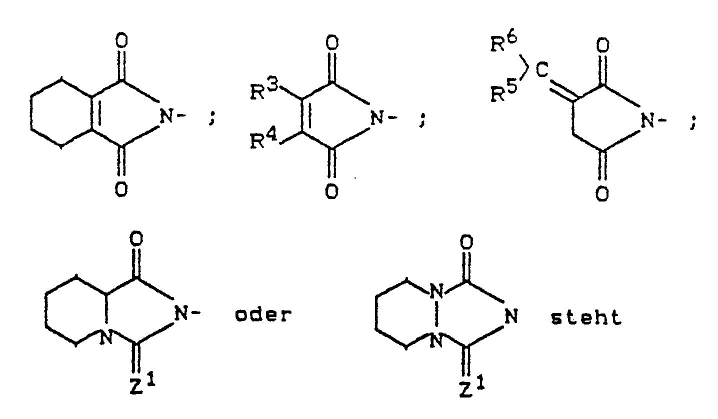 X¹ für Wasserstoff oder Fluor steht und
R¹ und R² unabhängig voneinander für Wasserstoff, Methyl oder Ethyl stehen,
wobei
Z¹ für Sauerstoff oder Schwefel steht,
R³ und R⁴ unabhängig voneinander jeweils für Wasserstoff oder Methyl stehen,
R⁵ und R⁶ entweder unabhängig voneinander jeweils für Wasserstoff oder Methyl oder gemeinsam für einen Ethan-1,2-diylrest, einen Butan-1,4-diylrest oder einen Pentan-1,5-diylrest stehen und
W für Sauerstoff oder eine NOR⁸-Gruppe steht
wobei
R⁸ für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen, insbesondere Fluor, Chlor oder Brom steht, für jeweils geradkettiges oder verzweigtes Cyanalkyl, Alkoxyalkyl, Alkoxyalkoxyalkyl oder Alkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor und Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, für jeweils gegebenenfalls durch Methyl oder Ethyl substituiertes Oxetanylmethyl, Oxetanylethyl, Tetrahydrofuranylmethyl oder Tetrahydropyranylmethyl steht oder für jeweils gegebenenfalls ein- bis dreifach gleich oder verschieden substituiertes Benzyl oder Phenylethyl steht, wobei als Substituenten jeweils in Frage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio.

5. Verfahren zur Herstellung von N-Aryl-Stickstoffheterocyclen der allgemeinen Formel (I), 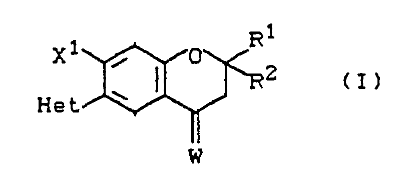 in welcher
X¹ für Wasserstoff oder Halogen steht,
R¹ und R² unabhängig voneinander jeweils für Wasserstoff oder Alkyl stehen und
Het für einen Heterocyclus der Formel 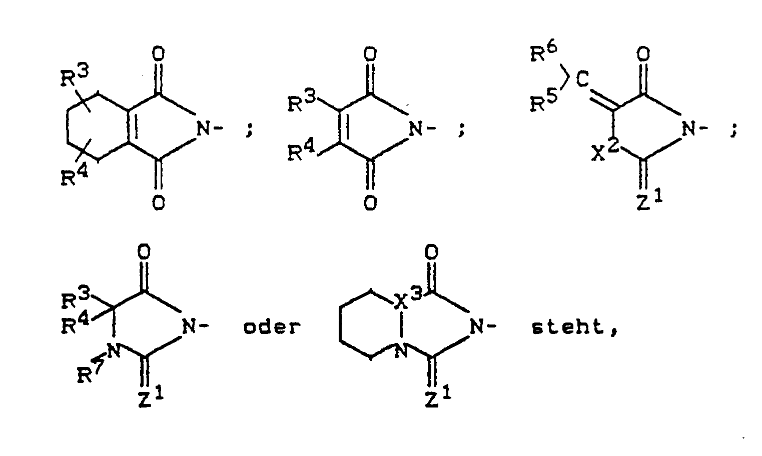 wobei
X² für Sauerstoff, für eine -CH₂-Gruppe oder für eine 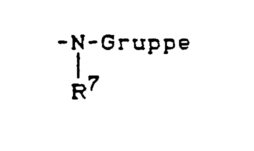 steht,
X³ für Stickstoff oder eine CH-Gruppe steht,
Z¹ für Sauerstoff oder Schwefel steht,
R³ und R⁴ unabhängig voneinander jeweils für Wasserstoff oder Alkyl stehen,
R⁵ und R⁶ entweder unabhängig voneinander jeweils für Wasserstoff oder Alkyl stehen oder gemeinsam für einen zweifach verknüpften Alkandiylrest stehen,
R⁷ für Wasserstoff, Alkyl oder für gegebenenfalls substituiertes Aryl steht und
W für Sauerstoff oder eine NOR⁸-Gruppe steht
wobei
R⁸ für Wasserstoff oder jeweils gegebenenfalls substituiertes Alkyl, Alkenyl, Alkinyl oder Cycloalkyl steht,
dadurch gekennzeichnet, daß man
(a) N-Aryl-Stickstoffheterocyclen der Formel (Ia), 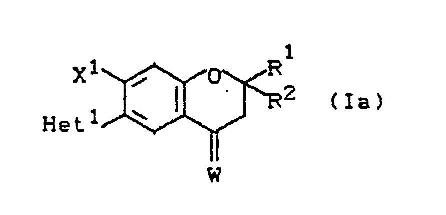 in welcher
Het¹ für einen Heterocyclus der Formel 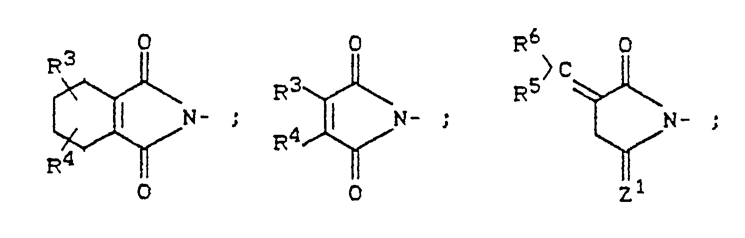 steht, wobei
Z¹, X¹, W, R¹, R², R³, R⁴, R⁵ und R⁶ die oben angegebene Bedeutung haben,
erhält, wenn man Anhydride der Formel (II), 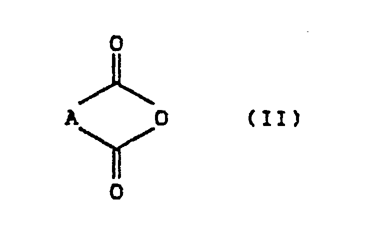 in welcher
A für einen Rest der Formel 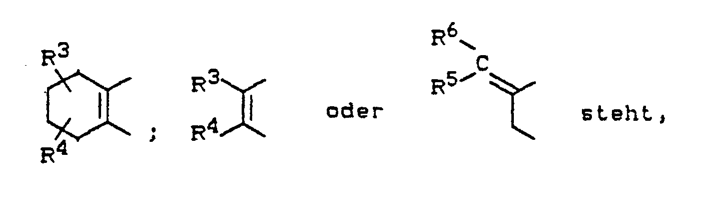 wobei
R³, R⁴, R⁵ und R⁶ die oben angegebene Bedeutung haben,
mit Aminen der Formel (III), 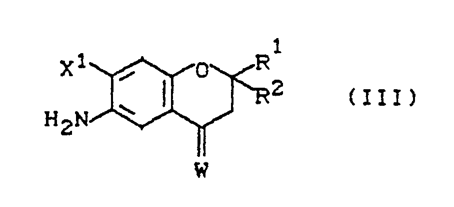 in welcher
X¹, R¹ und R² und W die oben angegebene Bedeutung haben,
gegebenenfalls in Gegenwart eines und gegebenenfalls in Gegenwart eines Reaktionshilfsmittels umsetzt; oder daß man
(b) N-Aryl-Stickstoffheterocyclen der Formel (Ib), 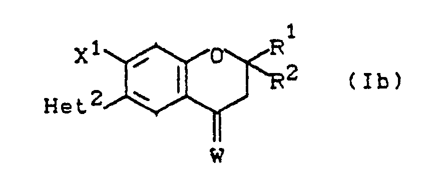 in welcher
Het² für einen Heterocyclus der Formel 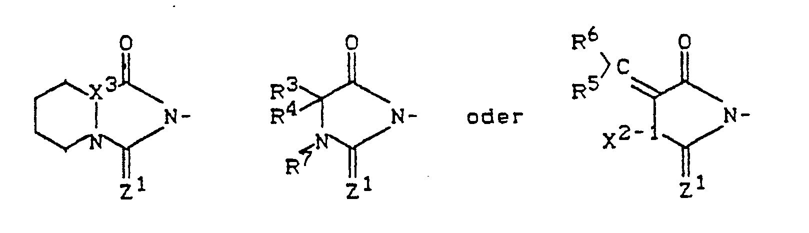 steht, wobei
X²⁻¹ für Sauerstoff oder eine 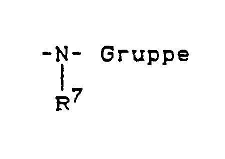 steht und
X¹, W, R¹, R², R³, R⁴, R⁵, R⁶, R⁷, X³ und Z¹ die oben angegebene Bedeutung haben,
erhält, wenn man Carbonsäureester der Formel (IV),
R⁹-COOR¹⁰ (IV)
in welcher
R⁹ für einen Rest der Formel 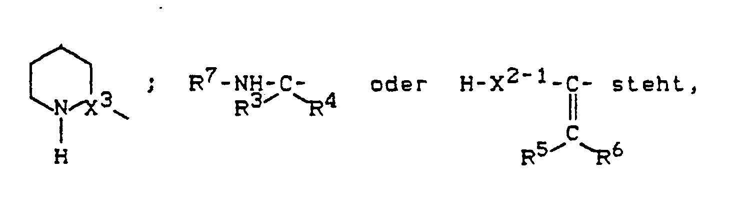
und
R¹⁰ für Alkyl steht, wobei
X²⁻¹, X³, R³, R⁴, R⁵, R⁶ und R⁷ die oben angegebene Bedeutung haben,
oder deren Säureadditionssalze mit Iso(thio)cyanaten der Formel (V), 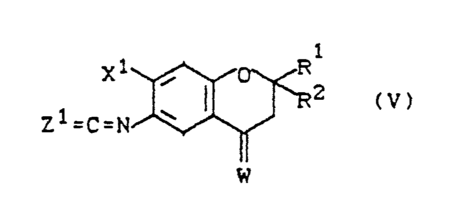 in welcher
Z¹, X¹, R¹, R² und W die oben angegebene Bedeutung haben,
gegebenenfalls in Gegenwart eines Verdünnungsmittels und gegebenenfalls in Gegenwart eines Reaktionshilfsmittels umsetzt; oder daß man
(c) N-Aryl-Stickstoffheterocyclen der Formel (Ic), 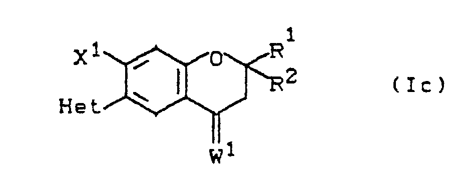 in welcher
W¹ für eine NOR⁸-Gruppe steht,
Het für einen Heterocyclus der Formel 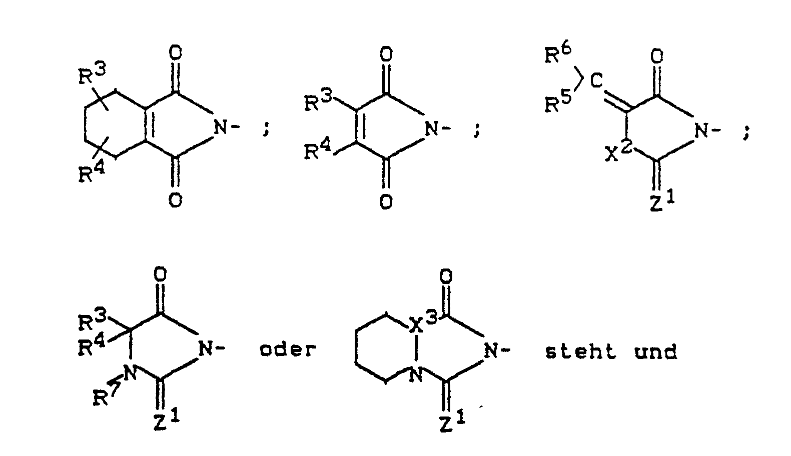 X¹, X², X³, Z¹, R¹, R², R³, R⁴, R⁵, R⁶, R⁷ und R⁸ die oben angegebene Bedeutung haben,
erhält, wenn man
(c-α) N-Aryl-Stickstoffheterocyclen der Formel (Id) 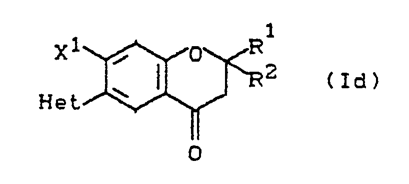 in welcher
Het, X¹, R¹ und R² die oben angegebene Bedeutung haben,
mit Hydroxylamin Derivaten der Formel (VI)
A x H₂N-OR⁸ (VI)
in welcher
R⁸ die oben angegebene Bedeutung hat und
A gegebenenfalls eine organische Säure darstellt,
gegebenenfalls in Gegenwart eines Verdünnungsmittels und gegebenfalls in Gegenwart eines Reaktionshilfsmittel umsetzt, oder
(c-β) N-Aryl-Stickstoffheterocyclen der Formel (Ie) 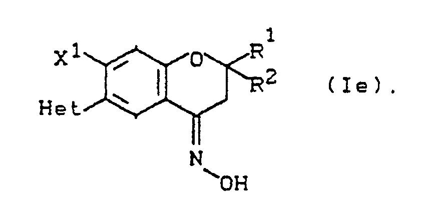 in welcher
Het, X¹, R¹ und R² die oben angegebene Bedeutung haben,
mit Alkylierungsmitteln der Formel (VII)
R⁸-E (VII)
in welcher
R⁸ die oben angegebene Bedeutung hat und
E für eine elektronenanziehende Abgangsgruppe steht,
gegebenenfalls in Gegenwart einer organischen oder anorganischen Base und eines Verdünnungsmittels umsetzt.

6. Herbizide Mittel, gekennzeichnet durch einen Gehalt an mindestens einem N-Aryl-Stickstoffheterocyclus der Formel (I) gemäß den Ansprüchen 1 bis 5.

7. Verfahren zur Bekämpfung von unerwünschten Pflanzen, dadurch gekennzeichnet, daß man N-Aryl-Stickstoffheterocyclen der Formel (I) gemäß den Ansprüchen 1 bis 5 auf die unterwünschten Pflanzen und/oder ihren Lebensraum einwirken läßt.

8. Verwendung von N-Aryl-Stickstoffheterocyclen der Formel (I) gemäß den Ansprüchen 1 bis 5 zur Bekämpfung von unerwünschten Pflanzen.

9. Verfahren zur Herstellung von herbiziden Mitteln, dadurch gekennzeichnet, daß man N-Aryl-Stickstoffheterocyclen der Formel (I) gemäß den Ansprüchen 1 bis 5 mit Streckmitteln und/oder oberflächenaktiven Substanzen vermischt.

10. Amine der allgemeinen Formel (IIIa) 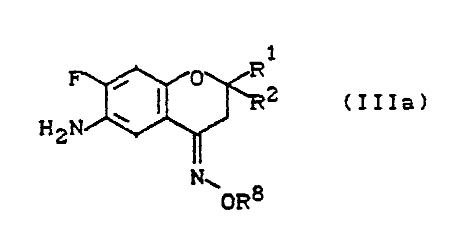 in welcher
R¹ und R² unabhängig voneinander jeweils für Wasserstoff oder Alkyl stehen und
R⁸ für Wasserstoff oder für jeweils gegebenenfalls substituiertes Alkyl, Alkenyl, Alkinyl oder Cycloalkyl steht.
